(19) Europäisches Patentamt
European Patent Office
Office européen des brevets

(11) Veröffentlichungsnummer: **0 205 021**
**A2**

(12) # EUROPÄISCHE PATENTANMELDUNG


(21) Anmeldenummer: 86107121.5

(22) Anmeldetag: 26.05.86

(51) Int. Cl.⁴: C 07 D 401/04
A 01 N 43/56

(30) Priorität: 07.06.85 DE 3520327

(43) Veröffentlichungstag der Anmeldung:
17.12.86 Patentblatt 86/51

(84) Benannte Vertragsstaaten:
AT BE CH DE FR GB IT LI NL SE



(71) Anmelder: BAYER AG
Konzernverwaltung RP Patentabteilung
D-5090 Leverkusen 1 Bayerwerk(DE)

(72) Erfinder: Dickoré, Karlfried, Dr.
Nicolai-Hartmann-Strasse 19
D-5090 Leverkusen 1(DE)

(72) Erfinder: Gehring, Reinhold, Dr.
Dasnöckel 49
D-5600 Wuppertal 11(DE)

(72) Erfinder: Sasse, Klaus, Dr.
Pützweg 13
D-5060 Bergisch-Gladbach 2(DE)

(72) Erfinder: Santel, Hans-Joachim, Dr.
Grünstrasse 9a
D-5090 Leverkusen 1(DE)

(72) Erfinder: Schmidt, Robert R., Dr.
Im Waldwinkel 110
D-5060 Bergisch-Gladbach 2(DE)


(54) **5-Amino-4-cyano-1-pyridyl-pyrazole.**


(57) Die Erfindung betrifft neue 5-Amino-4-cyano-1-pyridylpyrazole der Formel (I),

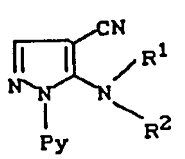
(I)

in welcher

$R^1$ für Wasserstoff, für jeweils gegebenenfalls substituiertes Alkyl, Alkenyl, Alkinyl oder Cycloalkyl oder für einen Rest

$-\underset{\underset{X}{\|}}{C}-R^3$ steht,

$R^2$ für Wasserstoff, für jeweils gegebenenfalls substituiertes Alkyl, Alkenyl, Alkinyl oder Cycloalkyl oder für einen Rest

$-\underset{\underset{X}{\|}}{C}-R^3$ steht, wobei

X für Sauerstoff oder Schwefel steht und für Wasserstoff, Alkyl, Alkoxy, Alkoxyalkyl, Alkylthio, Alkylthioalkyl, Halogenalkyl, Alkenyl, Alkinyl, für jeweils gegebenenfalls substituiertes Cycloalkyl, Aryl, Aryloxy, Arylthio oder Arylamino, für Alkylamino oder für Dialkylamino steht

oder

$R^1$ und $R^2$ gemeinsam mit dem Stickstoffatom, an welches sie gebunden sind, für einen gegebenenfalls substituierten Heterocyclus der Formel

stehen und

Py für substituiertes Pyridyl steht, aber nicht für den 5-Nitro-2-pyridyl- oder den 3-Chlor-5-trifluormethyl-2-pyridyl-Rest steht,

mehrere Verfahren zu ihrer Herstellung und ihre Verwendung als Herbizide.



EP 0 205 021 A2

Croydon Printing Company Ltd

BAYER AKTIENGESELLSCHAFT 5090 Leverkusen, Bayerwerk
Konzernverwaltung RP
Patentabteilung KM/li-c
Ib

5-Amino-4-cyano-1-pyridyl-pyrazole

Die Erfindung betrifft neue 5-Amino-4-cyano-1-pyridyl-pyrazole, mehrere Verfahren zu ihrer Herstellung und ihre Verwendung als Herbizide.

Es ist bereits bekannt, daß bestimmte 4-Cyano-1-aryl-pyrazole, wie beispielsweise das 4-Cyano-5-propionamido-1-(2,3,4-trichlorphenyl)-pyrazol, oder bestimmte 4-Cyano-1-pyridyl-pyrazole herbizide Eigenschaften besitzen (vergl. z. B. DE-OS 32 26 513, DE-OS 34 08 727).

Die herbizide Wirksamkeit dieser vorbekannten Verbindungen gegenüber Unkräutern ist jedoch ebenso wie ihre Verträglichkeit gegenüber wichtigen Kulturpflanzen nicht immer in allen Anwendungsgebieten völlig zufriedenstellend.

Weiterhin sind 5-Nitropyridyl-pyrazole bekannt (vgl. Rev. Latinoam Quim 13, 102- 103 (1982), welche als Zwischenprodukte bei der Herstellung von neuen heterocyclischen Systemen verwendet werden können. Eine Anwendung dieser Verbindungen in der Landwirtschaft ist jedoch nicht erwähnt.

Es wurden neue 5-Amino-4-cyano-1-pyridyl-pyrazole der allgemeinen Formel (I),

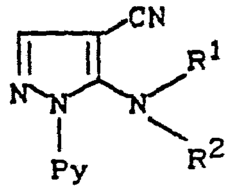

(I)

in welcher

$R^1$ für Wasserstoff, für jeweils gegebenenfalls substituiertes Alkyl, Alkenyl, Alkinyl oder Cycloalkyl oder für einen Rest $-\underset{\underset{X}{\|}}{C}-R^3$ steht,

$R^2$ für Wasserstoff, für jeweils gegebenenfalls substituiertes Alkyl, Alkenyl, Alkinyl oder Cycloalkyl oder für einen Rest $-\underset{\underset{X}{\|}}{C}-R^3$ steht, wobei

X für Sauerstoff oder Schwefel steht und

$R^3$ für Wasserstoff, Alkyl, Alkoxy, Alkoxyalkyl, Alkylthio, Alkylthioalkyl, Halogenalkyl, Alkenyl, Alkinyl, für jeweils gegebenenfalls substituiertes Cycloalkyl, Aryl, Aryloxy, Arylthio oder Arylamino, für Alkylamino oder für Dialkylamino steht

oder

$R^1$ und $R^2$ gemeinsam mit dem Stickstoffatom, an welches sie gebunden sind, für einen gegebenenfalls substituierten Heterocyclus der Formel

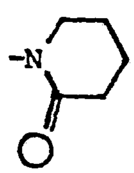 , 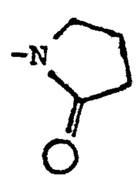 oder 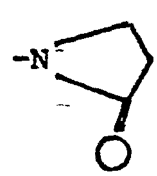 stehen und

Py für substituiertes Pyridyl steht, aber nicht für den 5-Nitro-2-pyridyl- oder den 3-Chlor-5-trifluormethyl-2-pyridyl-Rest steht,

gefunden.

Weiterhin wurde gefunden, daß sich die neuen 5-Amino-4-cyano-1-pyridyl-pyrazole der allgemeinen Formel (I)

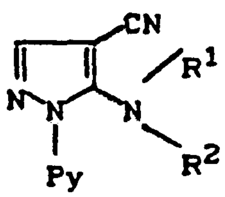

(I)

in welcher

$R^1$ für Wasserstoff, für jeweils gegebenenfalls substituiertes Alkyl, Alkenyl, Alkinyl oder Cycloalkyl oder für einen Rest $-\underset{\underset{X}{\|}}{C}-R^3$ steht,

$R^2$ für Wasserstoff, für jeweils gegebenenfalls substituiertes Alkyl, Alkenyl, Alkinyl oder Cycloalkyl oder für einen Rest $-\underset{\underset{X}{\|}}{C}-R^3$ steht,

wobei

X für Sauerstoff oder Schwefel steht und

$R^3$ für Wasserstoff, Alkyl, Alkoxy, Alkoxyalkyl, Alkylthio, Alkylthioalkyl, Halogenalkyl, Alkenyl, Alkinyl, für jeweils gegebenenfalls substituiertes Cycloalkyl, Aryl, Aryloxy, Arylthio oder Arylamino, für Alkylamino oder für Dialkylamino steht

oder

$R^1$ und $R^2$ gemeinsam mit dem Stickstoffatom, an welches sie gebunden sind, für einen gegebenenfalls substituierten Heterocyclus der Formel

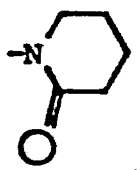 , 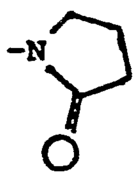 oder 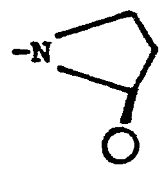

stehen und

Py für substituiertes Pyridyl steht, aber nicht für den 5-Nitro-2-pyridyl- oder den 3-Chlor-5-trifluormethyl-2-pyridyl-Rest steht,

nach folgenden Verfahren herstellen lassen:

Man erhält

(a) die erfindungsgemäßen 5-Amino-4-cyano-1-pyridyl-pyrazole der Formel (I),

wenn man

5-Halogen-4-cyano-1-pyridyl-pyrazole der Formel (II)

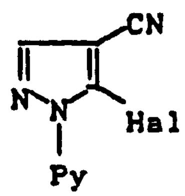

(II)

in welcher

Hal für Halogen steht und

Py die oben angegebene Bedeutung hat,

mit Aminoverbindungen der Formel (III),

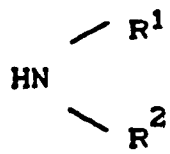

(III)

in welcher

$R^1$ und $R^2$ die oben angegebene Bedeutung haben,

gegebenenfalls in Gegenwart eines Verdünnungsmittels umsetzt,

oder man erhält

(b) die erfindungsgemäßen 5-Amino-4-cyano-1-pyridyl-pyrazol-Derivate der Formel (Ib),

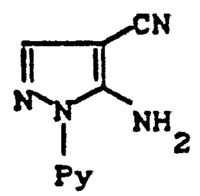

(Ib)

in welcher

Py die oben angegebene Bedeutung hat, wenn man

Pyridylhydrazine der Formel (IV)

$$Py\text{-}NH\text{-}NH_2 \qquad (IV)$$

in welcher

Py die oben angegebene Bedeutung hat,

mit Acrylnitrilderivaten der Formel (V),

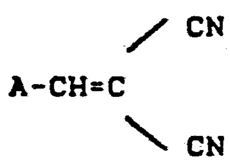

(V)

in welcher

A für Halogen, Hydroxy, Alkoxy oder Dialkylamino steht,

zunächst in einer 1. Stufe, gegebenenfalls in Gegenwart eines Verdünnungsmittels sowie gegebenenfalls in Gegenwart eines Reaktionshilfsmittels, umsetzt zu den Pyridylhydrazin-Derivaten der Formel (VI)

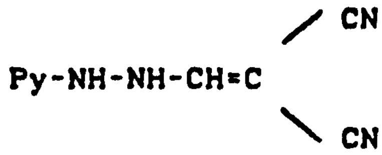

(VI)

in welcher

Py die oben angegebene Bedeutung hat,

und diese in einer 2. Stufe gegebenenfalls in Gegenwart eines Verdünnungsmittels und gegebenenfalls in Gegenwart eines Reaktionshilfsmittels cyclisiert,

oder man erhält

(c) die erfindungsgemäßen 5-Amino-4-cyano-1-pyridyl-pyrazol-Derivate der Formel (Ic),

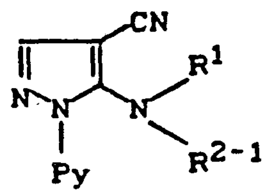

(Ic)

in welcher

$R^{2-1}$ für jeweils gegebenenfalls substituiertes Alkyl, Alkenyl, Alkinyl oder Cycloalkyl oder für einen Rest 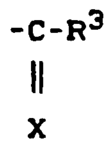 steht und

$R^1$, $R^3$, Py und X die oben angegebene Bedeutung haben.

wenn man

5-Amino-4-cyano-1-pyridyl-pyrazole der Formel (Ik),

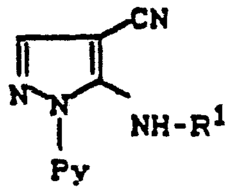

(Ik)

in welcher

$R^1$ und Py die oben angegebene Bedeutung haben, mit Verbindungen der Formel (VII)

$$R^{2-1}-A^1 \qquad (VII)$$

in welcher

$R^{2-1}$ für jeweils gegebenenfalls substituiertes Alkyl, Alkenyl, Alkinyl oder Cycloalkyl oder für einen Rest $-\underset{\underset{X}{\|}}{C}-R^3$ steht, wobei

$R^3$ und X die oben angegebene Bedeutung haben und

$A^1$ für eine elektronenziehende Abgangsgruppe steht,

gegebenenfalls in Gegenwart eines Verdünnungsmittels und gegebenenfalls in Gegenwart eines Säurebindemittels umsetzt,

oder man erhält

(d) die erfindungsgemäßen 5-Amino-4-cyano-1-pyridyl-pyrazole der Formel (Id)

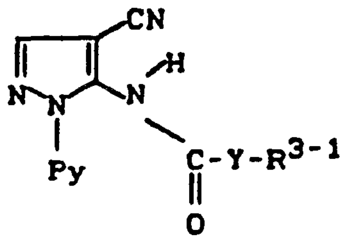

(Id)

in welcher

$R^{3-1}$ für Alkyl oder für gegebenenfalls substituiertes Aryl steht,

Y für Sauerstoff, Schwefel, eine (-NH-)- oder eine (>N-Alkyl)-Gruppe steht und

Py die oben angegebene Bedeutung hat,

wenn man

5-[N,N-Bis-(phenoxycarbonyl)-amino]-1-pyridyl-pyrazole der Formel (II)

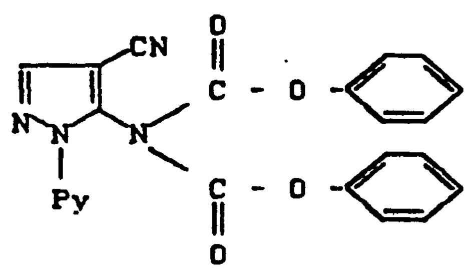

(II)

in welcher

Py die oben angegebene Bedeutung hat,

mit Verbindungen der Formel (VIII),

$R^{3-1}$-Y-H (VIII)

in welcher

$R^{3-1}$ für Alkyl oder für gegebenenfalls substituiertes Aryl steht und

Y für Sauerstoff, Schwefel, eine (-NH-)- oder eine (>N-Alkyl)-Gruppe steht,

gegebenenfalls in Gegenwart eines Verdünnungsmittels und gegebenenfalls in Gegenwart eines basischen Reaktionshilfsmittels umsetzt,

oder man erhält

(e) die erfindungsgemäßen 5-Amino-4-cyano-1-pyridyl-pyrazol-Derivate der Formel (Ie),

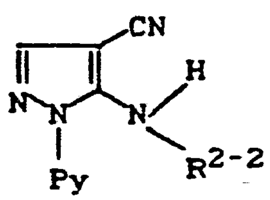

(Ie)

in welcher

$R^{2-2}$ für jeweils gegebenenfalls substituiertes Alkyl, Alkenyl, Alkinyl oder Cycloalkyl steht und

Py die oben angegebene Bedeutung hat,

wenn man

0205021

5-(N-Acylamino)-4-cyano-1-pyridyl-pyrazole der Formel (Im),

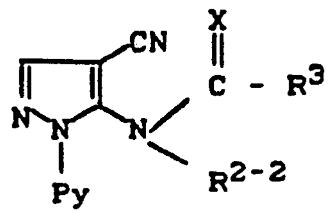

(Im)

in welcher

$R^3$, $R^{2-2}$, X und Py die oben angegebene Bedeutung haben,

mit Säuren oder Basen, gegebenenfalls in Gegenwart eines Verdünnungsmittels deacyliert,

oder man erhält

(f) die erfindungsgemäßen 5-Amino-4-cyano-1-pyridyl-pyrazol-Derivate der Formel (If),

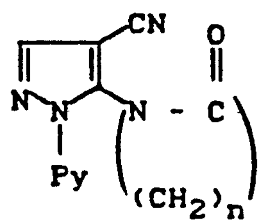

(If)

in welcher

n für 2, 3 oder 4 steht und

Py die oben angegebene Bedeutung hat,

wenn man

5-Halogenacylamino-4-cyano-pyrazole der Formel (In)

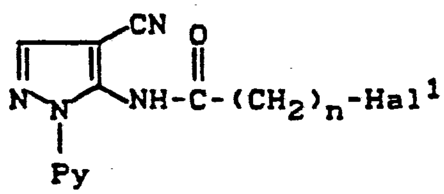

(In)

in welcher

$Hal^1$ für Halogen steht,

n für 2, 3 oder 4 steht und

Py die oben angegebene Bedeutung hat,

gegebenenfalls in Gegenwart eines Verdünnungsmittels und gegebenenfalls in Gegenwart eines basischen Reaktionshilfsmittels intramolekular cyclisiert,

oder man erhält

(g) die erfindungsgemäßen 5-Amino-4-cyano-1-pyridyl-pyrazol-Derivate der Formel (Ig),

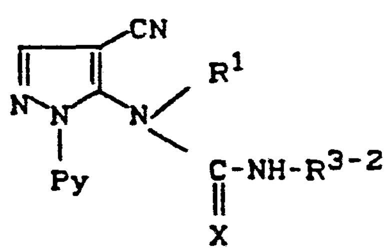

(Ig)

in welcher

$R^{3-2}$ für Alkyl oder für gegebenenfalls substituiertes Aryl steht,

X für Sauerstoff oder Schwefel steht und

$R^1$ und Py die oben angegebene Bedeutung haben,

wenn man

5-Amino-4-cyano-1-pyridyl-pyrazole der Formel (Ik),

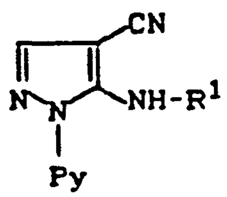

(Ik)

in welcher

$R^1$ und Py die oben angegebene Bedeutung haben,

mit Iso-(thio)-cyanaten der Formel (IX),

$$R^{3-2}\text{-N=C=X} \quad \text{(IX)}$$

in welcher

$R^{3-2}$ und X die oben angegebene Bedeutung haben,

gegebenenfalls in Gegenwart eines Verdünnungsmittels und gegebenenfalls in Gegenwart eines Reaktionshilfsmittels umsetzt.

Schließlich wurde gefunden, daß die neuen 5-Amino-4-cyano-1-pyridyl-pyrazole der Formel (I) herbizide, insbesondere auch selektiv-herbizide Eigenschaften besitzen.

Überraschenderweise zeigen die erfindungsgemäßen 5-Amino-4-cyano-1-pyridyl-pyrazole der allgemeinen Formel (I) eine bessere herbizide Wirksamkeit gegenüber Schadpflanzen, bei gleichzeitiger besserer Verträglichkeit gegenüber wichtigen Nutzpflanzen, als beispielsweise die aus dem Stand der Technik bekannten Verbindungen 4-Cyano-5-propionamido-1-(2,4,6-trichlorphenyl)- bzw. -(2,3,4-trichlorphenyl)-pyrazol, welche chemisch und wirkungsmäßig naheliegende Verbindungen sind.

Die erfindungsgemäßen 5-Amino-4-cyano-1-pyridyl-pyrazole sind durch die Formel (I) allgemein definiert.

Bevorzugt sind Verbindungen der Formel (I), bei welchen

$R^1$ für Wasserstoff, für jeweils gegebenenfalls einfach oder mehrfach, gleich oder verschieden substituiertes Alkyl mit 1 bis 8 Kohlenstoffatomen, Alkenyl oder Alkinyl mit jeweils 2 bis 8 Kohlenstoffatomen steht, wobei als Substituenten jeweils infrage kommen: Halogen, Cyano, Nitro, Hydroxy, Carboxy, jeweils geradkettiges oder verzweigtes Alkoxy oder Alkoxycarbonyl mit jeweils 1 bis 6 Kohlenstoffatomen im Alkylteil, Aminocarbonyl, welches gegebenenfalls durch Alkyl, Alkoxy, Alkylsulfonyl, Alkenyl oder Alkinyl

substituiert sein kann und wobei das Stickstoffatom des Aminocarbonylrestes auch Teil eines gesättigten 3- bis 7-gliedrigen Heterocyclus mit 1 oder 2 weiteren Heteroatomen, insbesondere Stickstoff, Sauerstoff oder Schwefel, sein kann;
für gegebenenfalls einfach oder mehrfach, gleich oder verschieden durch Halogen und/oder geradkettiges oder verzweigtes Alkyl mit 1 bis 4 Kohlenstoffatomen substituiertes Cycloalkyl mit 3 bis 7 Kohlenstoffatomen steht oder für einen Rest $-\underset{\underset{X}{\|}}{C}-R^3$ steht,

$R^2$ für Wasserstoff, für jeweils gegebenenfalls einfach oder mehrfach, gleich oder verschieden substituiertes Alkyl mit 1 bis 8 Kohlenstoffatomen, Alkenyl oder Alkinyl mit jeweils 2 bis 8 Kohlenstoffatomen steht, wobei als Substituenten jeweils infrage kommen: Halogen, Cyano, Nitro, Hydroxy, Carboxy, jeweils geradkettiges oder verzweigtes Alkoxy oder Alkoxycarbonyl mit jeweils 1 bis 6 Kohlenstoffatomen im Alkylteil, Aminocarbonyl, welches gegebenenfalls durch Alkyl, Alkoxy, Alkylsulfonyl, Alkenyl oder Alkinyl substituiert sein kann und wobei das Stickstoffatom des Aminocarbonylrestes auch Teil eines gesättigten 3- bis 7-gliedrigen Heterocyclus mit 1 oder 2 weiteren Heteroatomen, insbesondere Stickstoff, Sauerstoff oder Schwefel sein kann;

für gegebenenfalls einfach oder mehrfach, gleich oder verschieden durch Halogen und/oder geradkettiges oder verzweigtes Alkyl mit 1 bis 4 Kohlenstoffatomen substituiertes Cycloalkyl mit 3 bis 7 Kohlenstoffatomen steht oder für einen Rest $-\underset{\underset{X}{\|}}{C}-R^3$ steht,

wobei

X für Sauerstoff oder Schwefel steht,

$R^3$ für Wasserstoff, für jeweils geradkettiges oder verzweigtes Alkyl, Alkoxy, Alkoxyalkyl, Alkylthio oder Alkylthioalkyl mit jeweils 1 bis 4 Kohlenstoffatomen in den einzelnen Alkylteilen, für geradkettiges oder verzweigtes Halogenalkyl mit 1 bis 4 Kohlenstoffatomen und 1 bis 9 gleichen oder verschiedenen Halogenatomen, für jeweils geradkettiges oder verzweigtes Alkenyl oder Alkinyl mit jeweils 2 bis 4 Kohlenstoffatomen, für gegebenenfalls einfach oder mehrfach, gleich oder verschieden substituiertes Cycloalkyl, mit 3 bis 7 Kohlenstoffatomen, wobei als Substituenten Halogen, $C_1$-$C_4$-Alkyl oder Halogen-$C_1$-$C_4$-alkyl infrage kommen, für jeweils gegebenenfalls einfach oder mehrfach, gleich oder verschieden substituiertes Phenyl, Phenoxy, Phenylthio oder Phenylamino, wobei als Phenylsubstituenten Halogen, jeweils geradkettiges oder verzweigtes Alkyl oder Alkoxy mit jeweils 1 bis 4 Kohlenstoffatomen oder geradkettiges oder verzweigtes Halogenalkyl mit 1 bis 4 Kohlenstoffatomen und 1 bis 9 gleichen oder verschiedenen Halogenatomen infrage kommen, oder

für jeweils geradkettiges oder verzweigtes Alkylamino oder Dialkylamino mit jeweils 1 bis 4 Kohlenstoffatomen in den einzelnen Alkylteilen steht

oder

$R^1$ und $R^2$ gemeinsam mit dem Stickstoffatom, an welches sie gebunden sind, für einen jeweils gegebenenfalls einfach oder mehrfach, gleich oder verschieden durch geradkettiges oder verzweigtes Alkyl mit 1 bis 4 Kohlenstoffatomen substituierten Heterocyclus der Formel

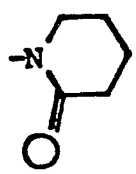 , 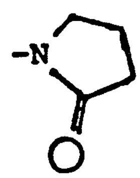 oder 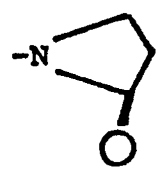

stehen und

Py für jeweils einfach oder mehrfach, gleich oder verschieden substituiertes 2-Pyridyl, 3-Pyridyl oder 4-Pyridyl steht, wobei als Substituenten jeweils infrage kommen:
Cyano, Nitro, Halogen, jeweils geradkettiges oder verzweigtes Alkyl, Alkoxy oder Alkoxycarbonyl mit jeweils 1 bis 4 Kohlenstoffatomen im Alkylteil, jeweils geradkettiges oder verzweigtes Halogenalkyl oder Halogenalkoxy mit jeweils 1 bis 4 Kohlenstoffatomen und jeweils 1 bis 9 gleichen oder verschiedenen Halogenatomen oder ein Rest $-S(O)_m-R^4$, wobei

$R^4$ für Amino, für jeweils geradkettiges oder verzweigtes Alkyl, Alkylamino, Dialkylamino mit jeweils 1 bis 4 Kohlenstoffatomen in den einzelnen Alkylteilen oder für geradkettiges oder verzweigtes Halogenalkyl mit 1 bis 4 Kohlenstoffatomen und mit 1 bis 9 gleichen oder verschiedenen Halogenatomen steht und

m für eine Zahl 0, 1 oder 2 steht,

Py aber nicht für den 5-Nitro-2-pyridyl-Rest oder den 3-Chlor-5-trifluormethyl-2-pyridyl-Rest steht.

Besonders bevorzugt sind Verbindungen der Formel (I), bei welchen

$R^1$ für Wasserstoff, für jeweils gegebenenfalls ein- bis dreifach, gleich oder verschieden substituiertes Methyl, Ethyl, n- oder i-Propyl, n-, i-, s- oder t-Butyl, Allyl, Propenyl, Butenyl, Propargyl oder Butinyl steht, wobei als Substituenten jeweils infrage kommen: Fluor, Chlor, Brom, Iod, Cyano, Nitro, Hydroxy, Carboxy, Methoxy, Ethoxy, n- oder i-Propoxy, Methoxycarbonyl, Ethoxycarbonyl, n- oder i-Propoxycarbonyl, n-, i-, s- oder t-Butoxycarbonyl, Aminocarbonyl, N-Methylaminocarbonyl, N-Ethylaminocarbonyl, N,N-Dimethylaminocarbonyl, N,N-Diethylaminocarbonyl, N-Methyl-N-ethylaminocarbonyl, N-Methyl-N-propylaminocarbonyl, N-Methyl- N-methoxyaminocarbonyl, N-Methylsulfonylaminocarbonyl, N-Ethylsulfonylaminocarbonyl, N,N-Diallylaminocarbonyl, N-Methyl-N-propargylaminocarbonyl, Pyrrolidin-1-ylcarbonyl, Piperidin-1-ylcarbonyl, Morpholin- 4-ylcarbonyl oder Perhydroazepin-1-ylcarbonyl:

für jeweils gegebenenfalls ein- bis fünffach, gleich oder verschieden durch Chlor, Methyl oder Ethyl substituiertes Cyclopropyl, Cyclopentyl, Cyclohexyl oder Cycloheptyl steht, oder für einen Rest $-\underset{\underset{X}{\|}}{C}-R^3$ steht,

$R^2$ für Wasserstoff, für jeweils gegebenenfalls ein- bis dreifach, gleich oder verschieden substituiertes Methyl, Ethyl, n- oder i-Propyl, n-, i-, s- oder t-Butyl, Allyl, Propenyl, Butenyl, Propargyl oder Butinyl steht, wobei als Substituenten jeweils infrage kommen: Fluor, Chlor, Brom, Iod, Cyano, Nitro, Hydroxy, Carboxy, Methoxy, Ethoxy, n- oder i-Propoxy, Methoxycarbonyl, Ethoxycarbonyl, n- oder i-Propoxycarbonyl, n-, i-, s- oder t-Butoxycarbonyl, Aminocarbonyl, N-Methylaminocarbonyl, N-Ethylaminocarbonyl, N,N-Dimethylaminocarbonyl, N,N-Diethylaminocarbonyl, N-Methyl-N-ethylaminocarbonyl, N-Methyl-N-propylaminocarbonyl, N-Methyl-N-methoxyaminocarbonyl, N-Methylsulfonylaminocarbonyl N-Ethylsulfonylaminocarbonyl, N,N-Diallylaminocarbonyl, N-Methyl-N-propargylaminocarbonyl, Pyrrolidin-1-ylcarbonyl, Piperidin-1-ylcarbonyl, Morpholin-4-ylcarbonyl oder Perhydroazepin-1-ylcarbonyl;

für jeweils gegebenenfalls ein- bis fünffach gleich oder verschieden durch Chlor, Methyl oder Ethyl substituiertes Cyclopropyl, Cyclopentyl, Cyclohexyl oder Cycloheptyl steht oder für einen Rest $-\underset{\underset{X}{\|}}{C}-R^3$ steht,

wobei

X für Sauerstoff oder Schwefel steht und

$R^3$ für Wasserstoff, Methyl, Ethyl, n- und i-Propyl, Methoxy, Ethoxy, Methoxymethyl, Ethoxymethyl, Methoxyethyl, Ethoxyethyl, Methylthio, Ethylthio, Methylthiomethyl, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 2-Bromethyl, Heptafluor-n-propyl, Allyl, Propenyl, Butenyl,Propargyl, für jeweils gegebenenfalls ein- bis vierfach, gleich oder verschieden durch Fluor, Chlor, Brom, Methyl oder Trifluormethyl substituiertes Cyclopropyl, Cyclopentyl oder Cyclohexyl, für jeweils gegebenenfalls ein- bis dreifach, gleich oder verschieden durch Methyl, Methoxy, Chlor oder Trifluormethyl substituiertes Phenyl, Phenoxy, Phenylthio oder Phenylamino steht, oder

$R^1$ und $R^2$ gemeinsam mit dem Stickstoffatom, an welches sie gebunden sind, für einen jeweils gegebenenfalls ein- bis vierfach, gleich oder verschieden durch Methyl, Ethyl, n- oder i-Propyl substituierten Heterocyclus der Formel

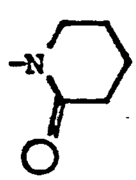 , 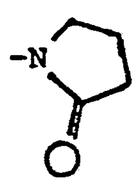 oder 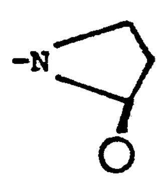

stehen und

Py für jeweils ein- bis vierfach, gleich oder verschieden substituiertes 2-Pyridyl, 3-Pyridyl oder 4-Pyridyl steht, wobei als Substituenten jeweils infrage kommen: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^4$, wobei

$R^4$ für Methyl, Ethyl, für Amino, Methylamino, Ethylamino, Dimethylamino, Diethylamino, Fluordichlormethyl, Difluormethyl, Tetrafluorethyl, Trifluorchlorethyl, Trichlormethyl, oder Trifluormethyl steht und

m für eine Zahl 0, 1 oder 2 steht,

Py aber nicht für den 5-Nitro-2-pyridyl-Rest oder den 3-Chlor-5-trifluormethyl-2-pyridyl-Rest steht.

Im einzelnen seien außer den bei den Herstellungsbeispielen genannten Verbindungen die folgenden 5-Amino-4-cyano-1-pyridyl-pyrazole der allgemeinen Formel (I) genannt:

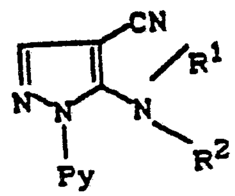

(I)

<u>Tabelle 1:</u>

| $R^1$ | $R^2$ | 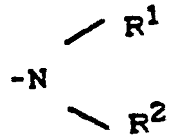 | Py |
|---|---|---|---|
| | | 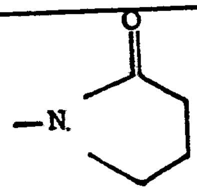 | 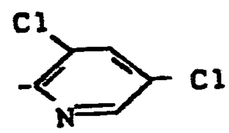 |
| | | 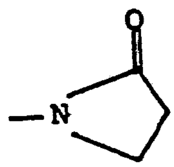 | 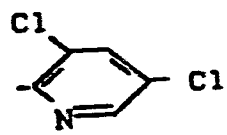 |
| H | $-CH_2-COOCH_3$ | | 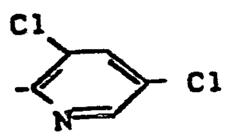 |
| H | $-\overset{O}{\overset{\|}{C}}-N(CH_3)_2$ | | 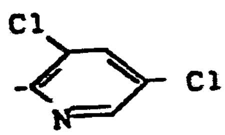 |
| H | $-\overset{O}{\overset{\|}{C}}-SCH_3$ | | 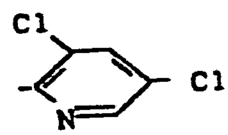 |
| H | $-\underset{CH_3}{CH}-COOC_2H_5$ | | 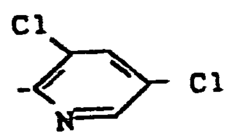 |

Verwendet man beispielsweise 5-Brom-4-cyano-1-(3,5-dichlor-4-pyridyl)-pyrazol und Diethylamin als Ausgangsstoffe, so läßt sich der Reaktionsablauf des erfindungsgemäßen Verfahrens (a) durch das folgende Formelschema darstellen:

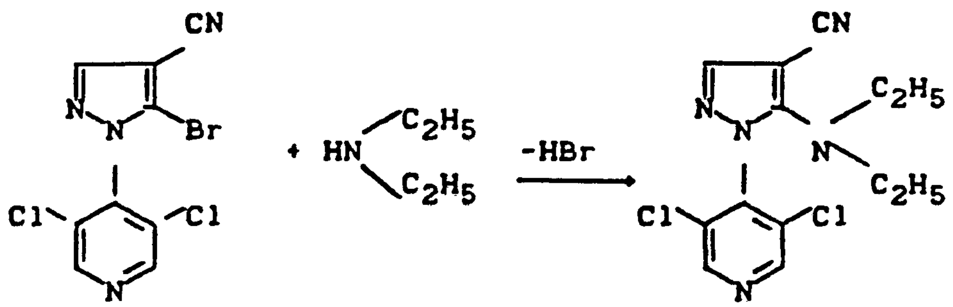


Verwendet man beispielsweise 3,5-Dichlor-2-pyridyl-hydrazin und Ethoxymethylenmalonsäuredinitril als Ausgangsstoffe, so läßt sich der Reaktionsablauf des erfindungsgemäßen Verfahrens (b) durch das folgende Formelschema darstellen:

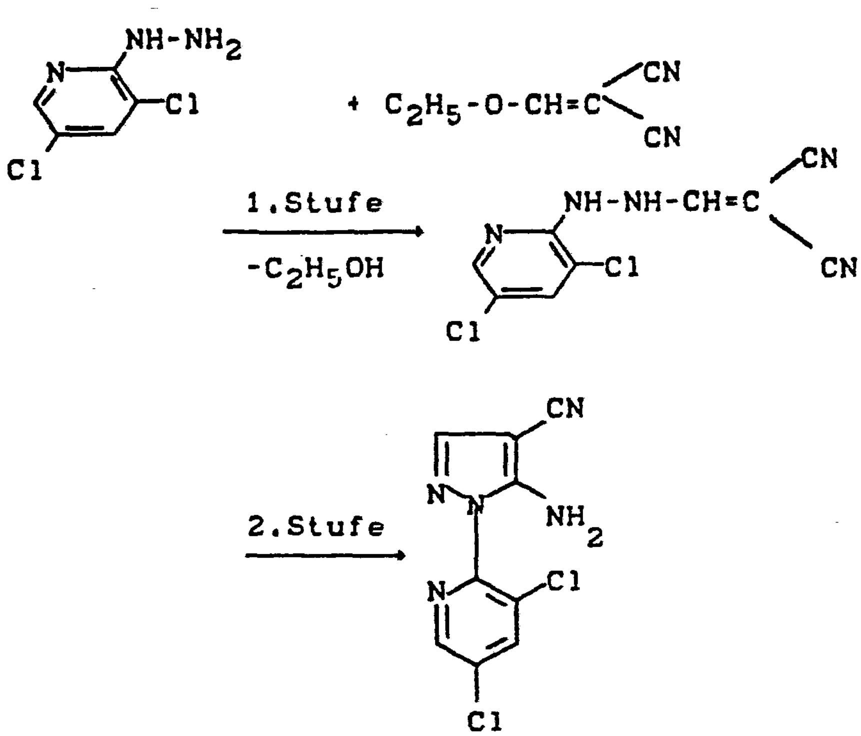


Verwendet man beispielsweise 5-Amino-4-cyano-1-(3,5-dichlor-2-pyridyl)-pyrazol und Propionylchlorid als Ausgangsstoffe, so läßt sich der Reaktionsablauf des erfindungsgemäßen Verfahrens (c) durch das folgende Formelschema darstellen:

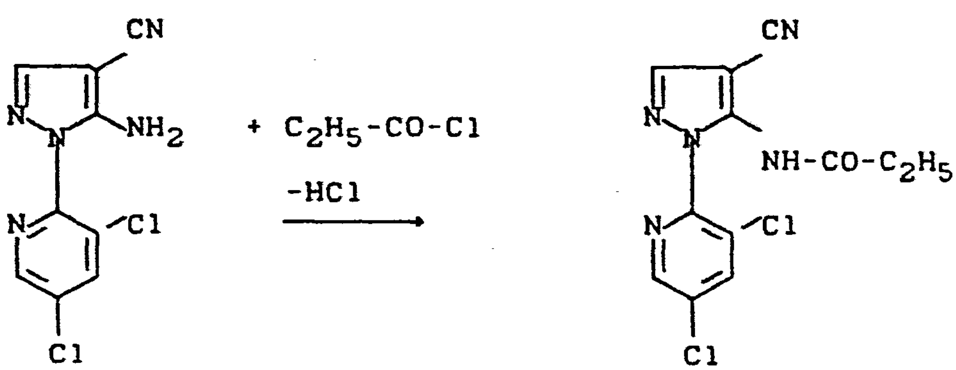

Verwendet man beispielsweise 5-[N,N-Bis(phenoxycarbonyl)-amino]-4-cyano-1-(3-chlor-5-trifluormethoxy-2-pyridyl)-pyrazol und Methanol als Ausgangsstoffe, so läßt sich der Reaktionsablauf des erfindungsgemäßen Verfahrens (d) durch das folgende Formelschema darstellen:

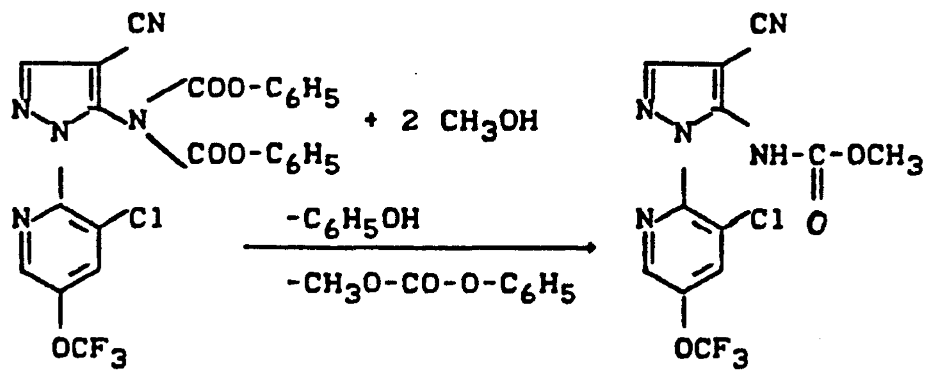


Verwendet man beispielsweise 5-(N-Butyl-N-propionamido)-4-cyano-1-(3-chlor-5-trifluormethylthio-2-pyridyl)-pyrazol als Ausgangsstoff, so läßt sich der Reaktionsablauf des erfindungsgemäßen Verfahrens (e) durch das folgende Formelschema darstellen:

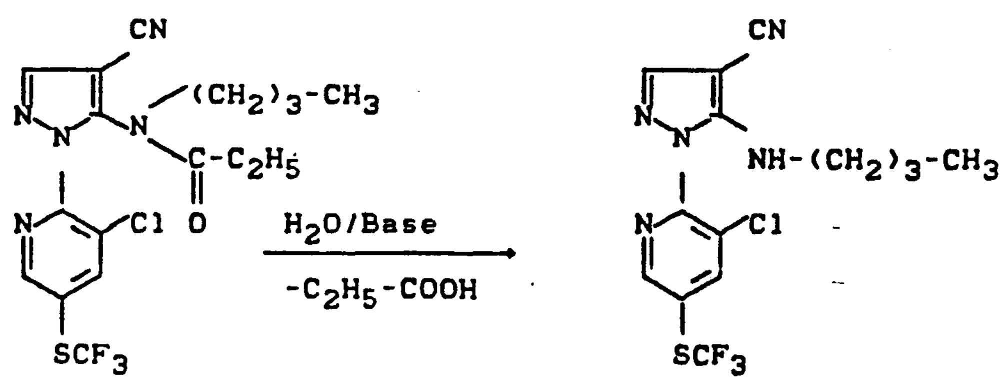

Verwendet man beispielsweise 5-(ω-chlorbutyramido)-4-cyano-1-(3,5-dibrom-2-pyridyl)-pyrazol als Ausgangsstoff, so läßt sich der Reaktionsablauf des erfindungsgemäßen Verfahrens (f) durch das folgende Formelschema darstellen:

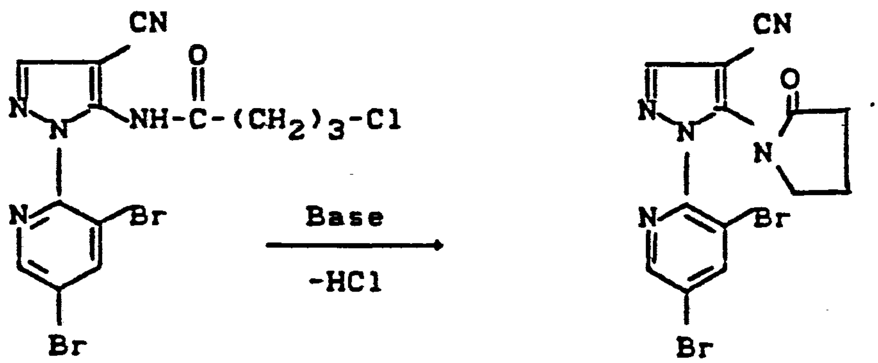


Verwendet man beispielsweise 5-Amino-4-cyano-1-(3,5-dichlor-2-pyridyl)-pyrazol und Methylisocyanat als Ausgangsstoffe, so läßt sich der Reaktionsverlauf des erfindungsgemäßen Verfahrens (g) durch das folgende Formelschema darstellen:

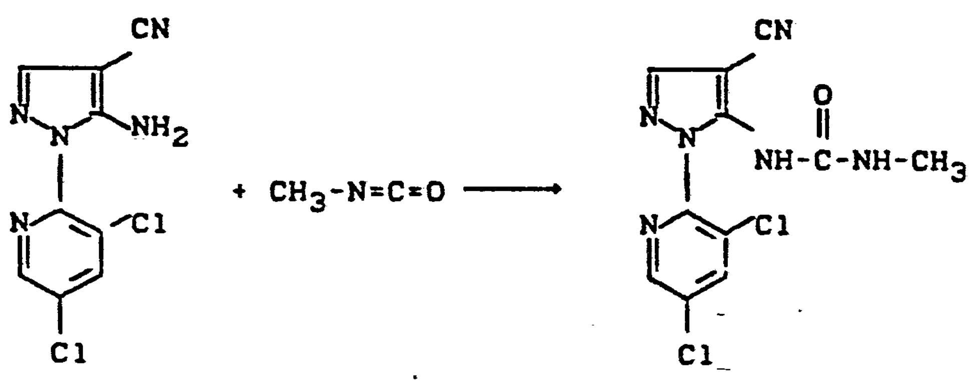

Die zur Durchführung des erfindungsgemäßen Verfahrens (a) als Ausgangsstoffe benötigten 5-Halogen-4-cyano-1-pyridyl-pyrazole sind durch die Formel (II) allgemein definiert. In dieser Formel (II) steht Py vorzugsweise für diejenigen Reste, die bereits im Zusammenhang mit der Beschreibung der erfindungsgemäßen Stoffe der Formel (I) als bevorzugt für diesen Substituenten genannt werden, Hal steht vorzugsweise für Chlor oder Brom.

Die 5-Halogen-4-cyano-1-pyridyl-pyrazole der Formel (II) sind noch nicht bekannt. Sie sind jedoch Gegenstand einer eigenen gleichzeitig eingereichten Patentanmeldung (DE-P 35 20 329 vom 7. Juni 1985).

Man erhält sie beispielsweise, wenn man Pyridylhydrazine der Formel (IV),

$$Py\text{-}NH\text{-}NH_2 \qquad (IV)$$

in welcher

Py die oben angegebene Bedeutung hat,

mit Ethoxymethylenmalonsäurediethylester der Formel (X)

$$C_2H_5\text{-}O\text{-}CH{=}C\begin{matrix} \diagup COOC_2H_5 \\ \diagdown COOC_2H_5 \end{matrix} \qquad (X)$$

gegebenenfalls in Gegenwart eines Verdünnungsmittels, wie beispielsweise Ethanol, bei Temperaturen zwischen 50° C und 200° C umsetzt, und die so erhältlichen 4-Ethoxycarbonyl-1-pyridyl-5-pyrazolone der Formel (XI),

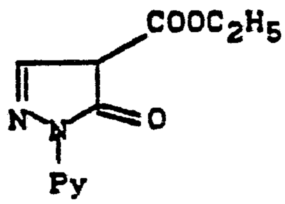

(XI)

in welcher

Py die oben angegebene Bedeutung hat,

in einer 2. Stufe mit Ammoniak gegebenenfalls in Gegenwart eines Verdünnungsmittels, wie beispielsweise Ethanol, bei Temperaturen zwischen 20°C und 100°C umsetzt und die so erhältlichen Pyrazol-Ammoniumsalze der Formel (XII),

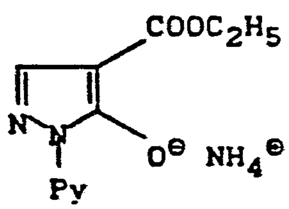

(XII)

in welcher

Py die oben angegebene Bedeutung hat,

in einer 3. Stufe mit einem Phosphoroxyhalogenid der Formel (XIII),

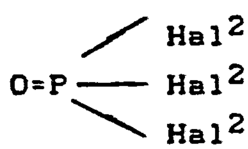

(XIII)

in welcher

$Hal^2$ für Halogen steht,

bei Temperaturen zwischen 100 und 200°C umsetzt.

Die Verbindungen der Formeln (X) und (XIII) sind allgemein bekannte Stoffe der organischen Chemie.

Die zur Durchführung des erfindungsgemäßen Verfahrens (a) weiterhin als Ausgangsstoffe benötigten Aminoverbindungen sind durch die Formel (III) allgemein definiert. In dieser Formel (III) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt werden, insbesondere stehen $R^1$ und $R^2$ für Wasserstoff, Alkyl, Alkenyl, Alkinyl oder Cycloalkyl.

Die Aminoverbindungen der Formel (III) sind allgemein bekannte Verbindungen der organischen Chemie.

Die zur Durchführung des erfindungsgemäßen Verfahrens (b) als Ausgangsstoffe benötigten Pyridylhydrazine sind durch die Formel (IV) allgemein definiert.

In dieser Formel (IV) steht Py vorzugsweise für diejenigen Reste, die bereits im Zusammenhang mit der Beschreibung der erfindungsgemäßen Stoffe der Formel (I) als bevorzugt für diesen Substituenten genannt werden.

Die Pyridylhydrazine der Formel (IV) sind bekannt [vergl. US-PS 4.127.575, US-PS 3.609.158, DE-OS 2 558 399, J.Chem. Soc.C, 167-174 (1971)] oder lassen sich nach prinzipiell bekannten Verfahren in einfacher analoger Weise herstellen, wenn man beispielsweise Halogenpyridine der Formel (XIV),

$$Py - Hal^3 \qquad (XIV)$$

in welcher

Py die oben angegebene Bedeutung hat und

$Hal^3$ für Halogen, insbesondere für Fluor, Chlor oder Brom steht,

mit Hydrazinhydrat gegebenenfalls in Gegenwart eines Verdünnungsmittels wie beispielsweise Pyridin oder Dioxan bei Temperaturen zwischen 0° und 150°C umsetzt, oder wenn man Aminopyridine der Formel (XV),

$$Py - NH_2 \qquad (XV)$$

in welcher

Py die oben angegebene Bedeutung hat,

in bekannter Weise, z. B. mit Natriumnitrit, in Gegenwart einer Säure wie beispielsweise Schwefelsäure diazotiert und anschließend ebenfalls in bekannter Weise die so erhältlichen Diazoniumsalze beispielsweise mit Zinn-II-chlorid in Gegenwart einer Säure, wie beispielsweise Salzsäure, bei Temperaturen zwischen -20°C und +80°C reduziert.

Die Halogenpyridine der Formel (XIV) und die Aminopyridine der Formel (XV) sind allgemein bekannte Verbindungen der organischen Chemie.

Die zur Durchführung des erfindungsgemäßen Verfahrens (b) weiterhin als Ausgangsstoffe benötigten Acrylnitrilderivate sind durch die Formel (V) allgemein definiert. In dieser Formel (V) steht A vorzugsweise für Chlor, Brom, Hydroxy, Methoxy, Ethoxy oder Dimethylamino.

Die Acrylnitrilderivate der Formel (V) sind bekannt [vergl. z. B.: DE-OS 31 29 429, DE-OS 32 06 878, EP 34 945, J.Chem.Soc. D, 1255 (1970), Can.J.Chem. 48, 2104-2109 (1970), J.Heterocyclic Chem. 19, 1267-1273 (1982), Can.J.Chem. 51, 1239-1244 (1973)] oder können nach bekannten Verfahren in einfacher analoger Weise erhalten werden.

Die zur Durchführung des erfindungsgemäßen Verfahrens (c) als Ausgangsstoffe benötigten 5-Amino-4-cyano-1-pyridyl-pyrazole sind durch die Formel (Ik) allgemein definiert.

In dieser Formel (Ik) stehen Py und $R^1$ vorzugsweise für diejenigen Reste, die bereits im Zusammenhang mit der Beschreibung der erfindungsgemäßen Stoffe der Formel (I) als bevorzugt für diese Substituenten genannt werden, besonders bevorzugt stehen Py und $R^1$ für diejenigen Reste, die ebenfalls bereits bei der Beschreibung der erfindungsgemäßen Stoffe der Formel (I) als besonders bevorzugt für diese Substituenten genannt werden.

Die 5-Amino-4-cyano-1-pyridyl-pyrazole der Formel (Ik) sind erfindungsgemäße Verbindungen. Die 5-Amino-4-cyano-1-pyridyl-pyrazole der Formel (Ik), bei welchen $R^1$ für Wasserstoff steht, sind erhältlich mit Hilfe der erfindungsgemäßen Verfahren (a) und (b). Die 5-Amino-4-cyano-1-pyridyl-pyrazole der Formel (Ik), bei welchen $R^1$ verschieden von Wasserstoff ist, sind erhältlich mit Hilfe der erfindungsgemäßen Verfahren (a), (d) und (g).

Außerdem können die nach dem Herstellungsverfahren (c) erhaltenen 5-Amino-4-cyano-1-pyridyl-pyrazole der Formel (Ic), in welcher $R^1$ für Wasserstoff steht, erneut als Ausgangsstoffe eingesetzt werden. Auf diese Weise lassen sich beispielsweise gemischt alkyl-acyl-substituierte Verbindungen herstellen.

Die zur Durchführung des erfindungsgemäßen Verfahrens (c) weiterhin als Ausgangsstoffe benötigten Verbindungen sind durch die Formel (VII) allgemein definiert. In dieser Formel (VII) steht $A^1$ vorzugsweise für Chlor, Brom, Iod, P-Toluolsulfonyloxy, Alkoxysulfonyloxy oder Acyloxy, $R^{2-1}$ steht vorzugsweise für jeweils gegebenenfalls einfach oder mehrfach, gleich oder verschieden substituiertes Alkyl mit 1 bis 8 Kohlenstoffatomen, Alkenyl oder Alkinyl mit jeweils 2 bis 8 Kohlenstoffatomen, wobei als Substituenten jeweils infrage kommen:

Halogen, Cyano, Nitro, Hydroxy, Carboxy, jeweils geradkettiges oder verzweigtes Alkoxy oder Alkoxycarbonyl mit jeweils 1 bis 6 Kohlenstoffatomen im Alkylteil, Aminocarbonyl, welches gegebenenfalls durch Alkyl, Alkoxy,

Alkylsulfonyl, Alkenyl oder Alkinyl substituiert sein kann und wobei das Stickstoffatom des Aminocarbonylrestes auch Teil eines gesättigten 3- bis 7-gliedrigen Heterocyclus mit 1 oder 2 weiteren Heteroatomen, insbesondere Stickstoff, Sauerstoff oder Schwefel, sein kann,

für gegebenenfalls einfach oder mehrfach, gleich oder verschieden durch Halogen oder geradkettiges oder verzweigtes Alkyl mit 1 bis 4 Kohlenstoffatomen substituiertes Cycloalkyl mit 3 bis 7 Kohlenstoffatomen oder für einen Rest

$$-\underset{\underset{X}{\|}}{C}-R^3,$$

wobei X und $R^3$ bevorzugt für diejenigen Substituenten stehen, die bereits bei der Beschreibung der erfindungsgemäßen Stoffe der Formel (I) als bevorzugt für X und $R^3$ genannt werden. In der Formel (VII) steht $R^{2-1}$ besonders bevorzugt für jeweils gegebenenfalls ein- bis dreifach, gleich oder verschieden substituiertes Methyl, Ethyl, n- oder i-Propyl, n-, i-, s- oder t-Butyl, Allyl, Propenyl, Butenyl, Propargyl oder Butinyl , wobei als Substituenten infrage kommen: Fluor, Chlor, Brom, Iod, Cyano, Nitro, Hydroxy, Carboxy, Methoxy, Ethoxy, n- oder i-Propoxy, Methoxycarbonyl, Ethoxycarbonyl, n- oder i-Propoxycarbonyl, n-, i-, s- oder t-Butoxycarbonyl, Aminocarbonyl, N-Methylaminocarbonyl, N-Ethylaminocarbonyl, N,N-Dimethylaminocarbonyl, N,N-Diethylaminocarbonyl, N-Methyl-N-ethylaminocarbonyl, N-Methyl-N-propylaminocarbonyl, N-Methyl-N-methoxyaminocarbonyl, N-Methylsulfonylaminocarbonyl, N-Ethylsulfonylaminocarbonyl, N,N-Diallylaminocarbonyl, N-Methyl-N-Propargylaminocarbonyl, Pyrrolidin-1-ylcarbonyl, Piperidin-1-ylcarbonyl, Morpholin-4-ylcarbonyl oder Perhydroazepin-1-ylcarbonyl, für jeweils gegebenenfalls ein- bis fünffach, gleich oder verschieden durch Chlor, Methyl oder Ethyl substituiertes Cyclopropyl, Cyclopentyl, Cyclohexyl oder Cycloheptyl steht, für einen Rest $-\underset{\underset{X}{\|}}{C}-R^3$,

wobei X und $R^3$ besonders bevorzugt für diejenigen Substituenten stehen, die bereits bei der Beschreibung der erfindungsgemäßen Stoffe der Formel (I) als besonders bevorzugt für X und $R^3$ genannt werden.

Die Verbindungen der Formel (VII) sind allgemein bekannte Verbindungen der organischen Chemie.

Die zur Durchführung des erfindungsgemäßen Verfahrens (d) als Ausgangsstoffe benötigten 5-[N,N-Bis-(phenoxycarbonyl)-amino]-1-pyridyl-pyrazole sind durch die Formel (II) allgemein definiert.

In dieser Formel (II) steht Py vorzugsweise für diejenigen Reste, die bereits im Zusammenhang mit der Beschreibung der erfindungsgemäßen Stoffe der Formel (I) als bevorzugt für diese Substituenten genannt werden.

Die 5-[N,N-Bis-(phenoxycarbonyl)-amino]-1-pyridyl-pyrazole der Formel (II) sind erfindungsgemäße Verbindungen und erhältlich mit Hilfe des erfindungsgemäßen Verfahrens (c).

Die zur Durchführung des erfindungsgemäßen Verfahrens (d) weiterhin als Ausgangsstoffe benötigten Verbindungen sind durch die Formel (VIII) allgemein definiert.

In dieser Formel (VIII) steht $R^{3-1}$ vorzugsweise für geradkettiges oder verzweigtes Alkyl mit 1 bis 4 Kohlenstoffatomen oder für gegebenenfalls einfach oder mehrfach, gleich oder verschieden substituiertes Phenyl, wobei als Substituenten infrage kommen: Halogen, jeweils geradkettiges oder verzweigtes Alkyl, Alkoxy mit jeweils 1 bis 4 Kohlenstoffatomen, für geradkettiges oder verzweigtes Halogenalkyl mit 1 bis 4 Kohlenstoffatomen und 1 bis 9 gleichen oder verschiedenen Halogenatomen.

$R^{3-1}$ steht insbesondere für Methyl, Ethyl oder für gegebenenfalls ein- bis dreifach, gleich oder verschieden durch Fluor, Chlor, Methyl, Methoxy oder Trifluormethyl substituiertes Phenyl. Y steht vorzugsweise für Sauerstoff, Schwefel, eine -NH-, -N-Methyl- oder -N-Ethyl-Gruppe.

Die Verbindungen der Formel (VIII) sind allgemein bekannte Verbindungen der organischen Chemie.

Die zur Durchführung des erfindungsgemäßen Verfahrens (e) als Ausgangsstoffe benötigten 5-(N-Acylamino)-4-cyano-1-pyridyl-pyrazole sind durch die Formel (Im) allgemein definiert.

In dieser Formel (Im) stehen $R^3$, X und Py vorzugsweise für diejenigen Reste, die bereits im Zusammenhang mit der Beschreibung der erfindungsgemäßen Stoffe der Formel (I)

als bevorzugt für diese Substituenten genannt werden.

$R^{2-2}$ steht vorzugsweise für jeweils gegebenenfalls einfach oder mehrfach, gleich oder verschieden substituiertes Alkyl mit 1 bis 8 Kohlenstoffatomen, Alkenyl oder Alkinyl mit jeweils 2 bis 8 Kohlenstoffatomen, wobei als Substituenten jeweils infrage kommen: Halogen, Cyano, Nitro, Hydroxy, Carboxy, jeweils geradkettiges oder verzweigtes Alkoxy oder Alkoxycarbonyl mit jeweils 1 bis 6 Kohlenstoffatomen im Alkylteil; außerdem für gegebenenfalls einfach oder mehrfach, gleich oder verschieden durch Halogen oder geradkettiges oder verzweigtes Alkyl mit 1 bis 4 Kohlenstoffatomen substituiertes Cycloalkyl mit 3 bis 7 Kohlenstoffatomen; insbesondere steht $R^{2-2}$ für jeweils gegebenenfalls ein- bis dreifach, gleich oder verschieden substituiertes Methyl, Ethyl, n- oder i-Propyl, n-, i-, s- oder t-Butyl, Allyl, Propenyl, Butenyl, Propargyl oder Butinyl, wobei als Substituenten jeweils infrage kommen: Fluor, Chlor, Brom, Iod, Cyano, Nitro, Hydroxy, Carboxy, Methoxy, Ethoxy, n- oder i-Propoxy; oder für jeweils gegebenenfalls ein- bis fünffach, gleich oder verschieden durch Chlor, Methyl oder Ethyl substituiertes Cyclopropyl, Cyclopentyl, Cyclohexyl oder Cycloheptyl.

Die 5-(N-Acylamino)-4-cyano-1-pyridyl-pyrazole der Formel (Im) sind erfindungsgemäße Verbindungen und erhältlich mit Hilfe des erfindungsgemäßen Verfahrens (c).

Die zur Durchführung des erfindungsgemäßen Verfahrens (f) als Ausgangsstoffe benötigten 5-Halogenacylamino-4-cyano-pyrazole sind durch die Formel (In) allgemein definiert.

In dieser Formel (In) steht Py vorzugsweise für diejenigen Reste, die bereits im Zusammenhang mit der Beschreibung der erfindungsgemäßen Stoffe der Formel (I) als bevorzugt für diesen Substituenten genannt werden. $Hal^1$ steht vorzugsweise für Chlor oder Brom und n steht vorzugsweise für eine Zahl 2, 3 oder 4.

Die 5-Halogenacylamino-4-cyano-pyrazole der Formel (In) sind erfindungsgemäße Verbindungen und erhältlich mit Hilfe des erfindungsgemäßen Verfahrens (c).

Die zur Durchführung des erfindungsgemäßen Verfahrens (g) als Ausgangsstoffe benötigten 5-Amino-4-cyano-1-pyridyl-pyrazole sind durch die Formel (Ik) definiert. In dieser Formel (Ik) stehen Py und $R^1$ vorzugsweise für diejenigen Substituenten, die bereits im Zusammenhang mit der Beschreibung der erfindungsgemäßen Stoffe der Formel (I) als bevorzugt für diese Reste genannt werden.

Die zur Durchführung des erfindungsgemäßen Verfahrens (g) weiterhin als Ausgangsstoffe benötigten Iso-(thio)-cyanate sind durch die Formel (IX) allgemein definiert.

In dieser Formel (IX) steht X vorzugsweise für Sauerstoff oder Schwefel, $R^{3-2}$ steht vorzugsweise für geradkettiges oder verzweigtes Alkyl mit 1 bis 4 Kohlenstoffatomen,

oder für gegebenenfalls ein bis dreifach, gleich oder verschieden substituiertes Phenyl, wobei als Substituenten infrage kommen: Halogen, jeweils geradkettiges oder verzweigtes Alkyl, Alkoxy mit jeweils 1 bis 4 Kohlenstoffatomen oder Halogenalkyl mit 1 bis 4 Kohlenstoffatomen und 1 bis 9 gleichen oder verschiedenen Halogenatomen.

$R^{3-2}$ steht insbesondere für Methyl, Ethyl und gegebenenfalls ein- bis dreifach, gleich oder verschieden durch Fluor, Chlor, Methyl, Methoxy oder Trifluormethyl substituiertes Phenyl.

Die Iso-(thio)-cyanate der Formel (IX) sind allgemein bekannte Verbindungen der organischen Chemie.

Zur Durchführung des erfindungsgemäßen Verfahrens (a) kommen als Verdünnungsmittel inerte organische Lösungsmittel infrage. Vorzugsweise verwendet man aliphatische, cyclische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether wie Diethylether, Diisopropylether, Dioxan, Tetrahydrofuran oder Ethylenglykoldiethylether oder -dimethylether, Ketone wie Aceton, Butanon, Methylisopropylketon oder Methylisobutylketon, Ester wie Essigsäureethylester, Nitrile, wie Acetonitril oder Propionitril, Amide wie Dimethylformamid, Dimethylacetamid, N-Methylpyrrolidon oder Hexamethylphosphorsäuretriamid. Es ist jedoch auch möglich, einen entsprechenden Überschuß der als Reaktionspartner eingesetzten Aminoverbindung der Formel (III) als Verdünnungsmittel zu verwenden.

Die Reaktionstemperaturen können bei der Durchführung des erfindungsgemäßen Verfahrens (a) in einem größeren Bereich variiert werden. Im allgemeinen arbeitet man bei Temperaturen zwischen -10°C und +120°C, vorzugsweise bei Temperaturen zwischen 0°C und 80°C.

Zur Durchführung des Verfahrens (a) setzt man pro Mol an 5-Halogen-4-cyano-1-pyridyl-pyrazol der Formel (II) im allgemeinen 1,0 bis 100,0 Mol, vorzugsweise 1,0 bis 50,0 Mol an Aminoverbindung der Formel (III) ein.

Zur Reaktionsdurchführung rührt man die Reaktionspartner, die gegebenenfalls in einem geeigneten Verdünnungsmittel gelöst werden, für mehrere Stunden bei der erforderlichen Reaktionstemperatur solange, bis im Dünnschichtchromatogramm kein Ausgangsprodukt mehr nachweisbar ist. Die Aufarbeitung erfolgt durch Entfernen der flüchtigen Bestandteile des Reaktionsgemisches im Vakuum, Aufnehmen des Rückstandes in einem mit Wasser nicht mischbaren organischen Lösungsmittel, Waschen mit Wasser, Trocknen und Entfernen des organischen Lösungsmittels im Vakuum.

Als Verdünnungsmittel zur Durchführung des Herstellungsverfahrens (b) kommen sowohl für die 1. als auch für die 2. Reaktionsstufe inerte organische Lösungsmittel infrage. Vorzugsweise verwendet man Alkohole, wie Methanol, Ethanol, Propanol, Butanol, Ethylenglykol oder Ethylenglykolmonomethyl- oder -ethylether.

Als Reaktionshilfsmittel zur Durchführung der 1. Stufe des Herstellungsverfahrens (b) kommen organische oder anorganische Säuren infrage. Vorzugsweise verwendet man Schwefelsäure oder Essigsäure, gegebenenfalls auch in Gegenwart einer Puffersubstanz, wie beispielsweise Natriumacetat.

Das Herstellungsverfahren (b) kann auch in einem Reaktionsschritt ohne Isolierung der Zwischenprodukte der Formel (VI) in Gegenwart eines Verdünnungsmittels durchgeführt werden.

Die Reaktionstemperaturen können bei der Durchführung der 1. Stufe des Herstellungsverfahrens (b) in gewissen Bereichen variiert werden. Im allgemeinen arbeitet man zwischen -30°C und +50°C, vorzugsweise zwischen -20°C und +20°C.

Als Reaktionshilfsmittel zur Durchführung der 2. Stufe des Herstellungsverfahrens (b) kommen alle üblicherweise verwendbaren Säuren, wie beispielsweise Schwefelsäure oder Phosphorsäure, infrage.

Die Reaktionstemperaturen können bei der Durchführung der 2. Stufe des Herstellungsverfahrens (b) ebenso wie bei der einstufigen Reaktionsführung in einem größeren Bereich variiert werden. Im allgemeinen arbeitet man zwischen 0°C und +200°C, vorzugsweise zwischen +50°C und 150°C.

Zur Durchführung des Herstellungsverfahrens (b) setzt man sowohl bei der einstufigen als auch bei der zweistufigen Reaktionsführung pro Mol Pyridylhydrazin der Formel (IV) im allgemeinen 1,0 bis 3,0 Mol, vorzugsweise 1,0 bis 1,5 Mol an Acrylnitril-Derivat der Formel (V) und im Fall des zweistufigen Verfahrens gegebenenfalls in der 1. Stufe

0205021

1,0 bis 10,0 Mol an Reaktionshilfsmittel und gegebenenfalls in der 2. Stufe ebenfalls 1,0 bis 10,0 Mol an Reaktionshilfsmittel ein.

Die Aufarbeitung und Isolierung der Reaktionsprodukte erfolgt nach üblichen Verfahren, beispielsweise durch Entfernen des organischen Verdünnungsmittels, Ausfällen des Reaktionsproduktes in Wasser, Absaugen und Trocknen des so erhaltenen Produktes.

Zur Durchführung des Herstellungsverfahrens (c) kommen als Verdünnungsmittel ebenfalls inerte organische Lösungsmittel infrage. Vorzugsweise verwendet man die bei Verfahren (a) genannten Lösungsmittel.

Es ist jedoch auch möglich, die als Reaktionspartner verwendeten Verbindungen der Formel (VII) in entsprechendem Überschuß als Verdünnungsmittel einzusetzen.

Als Säurebindemittel zur Durchführung des Herstellungsverfahrens (c) kommen alle üblicherweise verwendbaren anorganischen und organischen Basen infrage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des Herstellungsverfahrens (c) in einem größeren Bereich variiert werden. Im allgemeinen arbeitet man zwischen

-20°C und +150°C, vorzugsweise zwischen 0°C und +100°C.

Zur Durchführung des Herstellungsverfahrens (c) setzt man pro Mol 5-Amino-1-pyridyl-pyrazol der Formel (Ik) im allgemeinen 1,0 bis 20,0 Mol, vorzugsweise 1,0 bis 15,0 Mol an Verbindung der Formel (VII) und gegebenenfalls 1,0 bis 3,0 Mol, vorzugsweise 1,0 bis 2,0 Mol an Säurebindemittel ein. Die Reaktionsdurchführung, Aufarbeitung und Isolierung der Reaktionsprodukte der Formel (Ic) erfolgt in allgemein üblicher Art und Weise.

Als Verdünnungsmittel zur Durchführung des erfindungsgemäßen Verfahrens (d) kommen ebenfalls inerte organische Lösungsmittel infrage. Vorzugsweise verwendet man die bei Verfahren (a) genannten organischen Lösungsmittel oder Alkohole, wie Methanol, Ethanol oder Isopropanol.

Es ist jedoch auch möglich, die als Reaktionskomponenten verwendeten Alkohole, Amine oder Thiole der Formel (VIII) in entsprechendem Überschuß gleichzeitig als Verdünnungsmittel einzusetzen.

Die Reaktionstemperaturen können bei dem erfindungsgemäßen Verfahren (d) ebenfalls in einem größeren Bereich variiert werden. Im allgemeinen arbeitet man zwischen 0°C und +200°C, vorzugsweise zwischen +20°C und +150°C.

Das erfindungsgemäße Verfahren (d) kann gegebenenfalls in Gegenwart eines basischen Reaktionshilfsmittels durchgeführt werden. Als solche kommen alle üblicherweise verwendbaren Basen, wie beispielsweise die bei Verfahren (c) genannten, infrage.

Zur Durchführung des erfindungsgemäßen Verfahrens (d) setzt man pro Mol 5-[N,N-Bis-(phenoxycarbonyl)-amino]-1-pyridyl-pyrazol der Formel (II) im allgemeinen 1 bis 20 Mol, vorzugsweise 1 bis 10 Mol Verbindung der Formel (VIII) und gegebenenfalls 1 bis 10 Mol an Base ein und erwärmt für mehrere Stunden auf die erforderliche Temperatur. Die Aufarbeitung und Isolierung der Reaktionsprodukte der Formel (Id) erfolgt nach üblichen Verfahren.

Als Verdünnungsmittel zur Durchführung des erfindungsgemäßen Verfahrens (e) kommen inerte organische oder anorganische Lösungsmittel infrage.

Insbesondere seien die bei Verfahren (d) aufgezählten organischen Lösungsmittel genannt. Besonders bevorzugt sind Alkohole wie Methanol oder Ethanol oder deren Gemische mit Wasser.

Das Verfahren (e) wird entweder in Gegenwart einer starken Säure, wie beispielsweise Salzsäure, Trifluoressigsäure oder Bromwasserstoffsäure, in Eisessig oder in Gegenwart einer Base durchgeführt. Als Basen sind wässrige Lösungen von Natriumhydroxid oder Kaliumhydroxid bevorzugt.

Die Reaktionstemperaturen können bei der Durchführung des erfindungsgemäßen Verfahrens (e) in einem größeren Bereich variiert werden. Im allgemeinen arbeitet man bei Temperaturen zwischen -20°C und +150°C, vorzugsweise bei Temperaturen zwischen 0°C und +120°C.

Zur Durchführung des erfindungsgemäßen Verfahrens (e) setzt man pro Mol 5-(N-Acylamino)-4-cyano-1-pyridyl-pyrazol der Formel (Im) im allgemeinen 1 bis 30 Mol, vorzugsweise 1 bis 15 Mol, an Säure oder Base ein. Die Reaktionsdurchführung, Aufarbeitung und Isolierung der Reaktionsprodukte der Formel (Ie) erfolgt in allgemein üblicher Art und Weise.

Als Verdünnungsmittel zur Durchführung des erfindungsgemäßen Verfahrens (f) kommen ebenfalls inerte organische Lösungsmittel infrage, vorzugsweise verwendet man die bei Verfahren (d) aufgezählten Lösungsmittel.

Als basische Reaktionshilfsmittel kommen bei der Durchführung des erfindungsgemäßen Verfahrens (f) alle üblichen anorganischen oder organischen Basen infrage. Vorzugsweise verwendet man Alkalimetallcarbonate wie Kaliumcarbonat oder Kaliumhydrogencarbonat, Alkalimetallhydroxide oder -hydride, wie Natriumhydroxid oder Natriumhydrid, oder Alkoholate, wie Natriummethylat oder -ethylat.

Die Reaktionstemperaturen können bei der Durchführung des erfindungsgemäßen Verfahrens (f) in einem größeren Bereich variiert werden. Im allgemeinen arbeitet man bei Temperaturen zwischen 0°C und 130°C, vorzugsweise bei Temperaturen zwischen 20°C und 100°C.

Zur Durchführung des erfindungsgemäßen Verfahrens (f) setzt man pro Mol an 5-Halogenacylamino-4-cyano-pyrazol der Formel (In) im allgemeinen 1,0 bis 3,0 Mol, insbesondere 1,0 bis 2,0 Mol an basischem Reaktionshilfsmittel ein. Die Reaktionsdurchführung, Aufarbeitung und Isolierung der Reaktionsprodukte der Formel (If) erfolgen in Analogie zu bekannten Verfahren (vergl. z.B. DE-OS 33 25 488).

Zur Durchführung des Herstellungsverfahrens (g) kommen als Verdünnungsmittel ebenfalls inerte organische Lösungsmittel infrage. Vorzugsweise verwendet man aliphatische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether wie Diethylether, Diisopropylether, Dioxan, Tetrahydrofuran oder Ethylenglykoldiethylether oder -dimethylether, Ketone wie Aceton, Butanon, Methylisopropylketon oder Methylisobutylketon, Ester wie Essigsäureethylester, Nitrile wie Acetonitril oder Propionitril, Amide wie Dimethylformamid, Dimethylacetamid, N-Methylpyrrolidon oder Hexamethylphosphorsäuretriamid. Verwendet man Iso-(thio)-cyanate der Formel (IX) in flüssiger Form, so ist es auch möglich, diese in entsprechendem Überschuß als Verdünnungsmittel einzusetzen.

Als Reaktionshilfsmittel zur Durchführung des Herstellungsverfahrens (g) kommen alle üblicherweise verwendbaren anorganischen und organischen Basen infrage. Vorzugsweise verwendet man Alkalimetallcarbonate oder -hydrogencarbonate wie beispielsweise Natriumcarbonat oder Natriumhydrogencarbonat oder auch tertiäre Amine wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des Herstellungsverfahrens (g) in einem größeren Bereich variiert werden. Im allgemeinen arbeitet man zwischen -20°C und +150°C, vorzugsweise zwischen 0°C und +100°C.

Zur Durchführung des Herstellungsverfahrens (g) setzt man pro Mol 5-Amino-4-cyano-1-pyridyl-pyrazol der Formel (Ik) im allgemeinen 1,0 bis 20,0 Mol, vorzugsweise 1,0 bis 15,0 Mol an Verbindung der Formel (IX) und gegebenenfalls 1,0 bis 3,0 Mol, vorzugsweise 1,0 bis 2,0 Mol an Reaktionshilfsmittel ein. Die Reaktionsdurchführung, Aufarbeitung und Isolierung der Reaktionsprodukte der Formel (Ig) erfolgt in allgemein üblicher Art und Weise.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia,

Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe der Formel (I) mit besonders gutem Erfolg zur selektiven Bekämpfung breitblättriger Unkräuter in mono- und dikotylen Kulturen wie Baumwolle, Weizen oder Mais einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z. B. auch organische Lösungsmittel als Hilfslösungsmittel verwendet werden. Als flüssige Lösungsmittel kommen im wesentlichen infrage: Aromaten wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe wie Cyclohexan oder Paraffine, z. B. Erdölfraktionen, mineralische und pflanzliche Öle, Alkohole wie Butanol oder Glycol sowie deren Ether und Ester, Ketone wie Aceton, Methylethylketon, Methylisobutylketon oder Cyclohexanon, stark polare Lösungsmittel wie Dimethylformamid und Dimethylsulfoxid, sowie Wasser.

Als feste Trägerstoffe kommen infrage: z. B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen infrage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln: als Emulgier- und/ oder schaumerzeugende Mittel kommen infrage: z. B. nichtionogene und anionische Emulgatoren, wie Polyoxyethylen-Fettsäure-Ester, Polyoxyethyl-Fettalkohol-Ether, z. B. Alkylaryl-polyglykolether, Alkylsulfonate, Alkylsulfate, Arylsulfonate sowie Eiweißhydrolysate: als Dispergiermittel kommen infrage: z. B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z. B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen infrage.

Auch Mischungen mit N,N-Dimethyl-N'-(3-chlor-4-methylphenyl)-harnstoff, N,N-Dimethyl-N'-(4-isopropylphenyl)-harnstoff, 4-Amino-6-t-butyl-3-ethylthio-1,2,4-triazin-5(4H)-on, 2,4-Dichlorphenoxyessigsäure, 2,4-Dichlorphenoxypropionsäure, (2-Methyl-4-chlorphenoxy)-essigsäure, (4-Chlor-2-methylphenoxy)-propionsäure, Chloressigsäure-N-(methoxymethyl)-2,6-diethylanilid, 2-Ethyl-6-methyl-N-(1-methyl-2-methoxyethyl)-chloracetanilid, 2,6-Dinitro-4-trifluormethyl-N,N-dipropylanilin, 2-[4-(3,5-Dichlorpyrid-2-yloxy)-phenoxy]-propionsäure-(2-benzyloxyethylester), -(trimethylsilylmethylester) oder -(2,2-diethoxyethylester), N,N-Diisopropyl-S-(2,3,3-trichlorallyl)-thiolcarbamat, [(4-Amino-3,5-dichlor-6-fluor-2-pyridinyl)-oxy]-essigsäure bzw.

-1-methylheptylester, 3,5-Dibrom-4-hydroxybenzonitril, Methyl-5-(2,4-dichlorphenoxy)-2-nitrobenzoat, 3,5-Diiod-4-hydroxybenzonitril, 3-Isopropyl-2,1,3-benzothiadiazin-4-on-2,2-dioxid, 2-Chlor-N-{[(4-methoxy-6-methyl-1,3,5-triazin-2-yl)-amino]-carbonyl}-benzolsulfonamid, N,N-Dimethyl-N'-(3-trifluormethylphenyl)-harnstoff, N-Methyl-2-(1,3-benzthiazol-2-yloxy)-acetanilid, 2-Chlor-4-ethylamino-6-isopropylamino-1,3,5-triazin, exo-1-Methyl-4-(1-methylethyl)-2-(2-methylphenylmethoxy)-7-oxabicyclo-[2.2.1]-heptan, 2-{4-[[3-Chlor-5-(trifluormethyl)-2-pyridinyl]-oxy]-phenoxy}-propansäure bzw. -propansäureethylester, N-(1-Ethylpropyl)-3,4-dimethyl-2,6-dinitroanilin oder 6-Chlor-3-phenyl-pyridazin-4-yl-S-octyl-thiocarbonat sind von Vorteil. Einige Mischungen zeigen überraschenderweise auch synergistische Wirkung.

Auch eine Mischung mit anderen bekannten Wirkstoffen wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1:

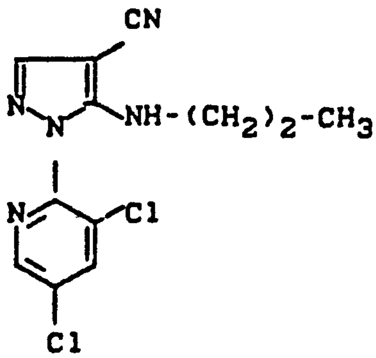

[Verfahren (a)]

3,2 g (0.01 Mol) 5-Brom-4-cyano-1-(3,5-dichlorpyrid-2-yl)-pyrazol und 30 ml (0.36 Mol) n-Propylamin werden für 15 Stunden bei Raumtemperatur gerührt, im Vakuum eingeengt, der Rückstand in Chloroform aufgenommen, zweimal mit Wasser gewaschen, über Natriumsulfat getrocknet und im Vakuum vom Lösungsmittel befreit.

Man erhält 2.2 g (75 % der Theorie) an 4-Cyano-5-n-propylamino-1-(3,5-dichlorpyrid-2-yl)-pyrazol vom Schmelzpunkt 90°C.

Herstellung der Ausgangsverbindung

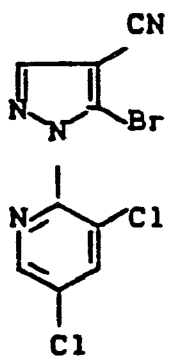

Zu 12,7 g (0.05 Mol) 5-Amino-4-cyano-1-(3,5-dichlorpyrid-2-yl)-pyrazol in 100 ml Bromwasserstoffsäure gibt man bei -5° bis 0°C 6 g (0.087 Mol) Natriumnitrit in 15 ml Wasser und rührt 5 Stunden bei Raumtemperatur. Der ausgefallene Niederschlag wird abgesaugt, in Wasser angeschlämmt, mit Natriumhydrogencarbonat schwach alkalisch gestellt, wiederum abgesaugt und getrocknet.

Man erhält 11 g (70 % der Theorie) an 5-Brom-4-cyano-1-(3,5-dichlorpyrid-2-yl)-pyrazol vom Schmelzpunkt 142°C (Zersetzung).

Beispiel 2:

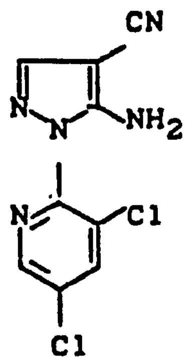

[Verfahren (b)]

24,2 g (0.136 Mol) N-(3,5-Dichlorpyrid-2-yl)-hydrazin und 16.6 g (0.136 Mol) Ethoxymethylenmalonsäuredinitril in 200 ml Ethanol werden für 15 Stunden unter Rückfluß erhitzt. Der aus der erkalteten Reaktionsmischung ausgefallene Niederschlag wird abgesaugt und getrocknet.

Man erhält 28.8 g (83 % der Theorie) an 5-Amino-4-cyano-1- (3,5-dichlor-2-pyridyl)-pyrazol vom Schmelzpunkt 213° - 215°C.

Herstellung der Ausgangsverbindung:

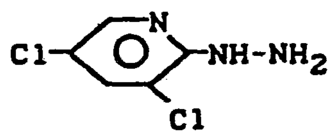

34,0 g (0.186 Mol) 2,3,5-Trichlorpyridin und 51 g (1.0 Mol) Hydrazinhydrat in 400 ml Dioxan werden für 3 Stunden unter Rückfluß erhitzt und der aus der erkalteten Reaktionsmischung ausgefallene Niederschlag abgesaugt und getrocknet. Man erhält 24.2 g (73 % der Theorie) an N-(3,5-Dichlorpyrid-2-yl)-hydrazin vom Schmelzpunkt 179°-182°C.

Beispiel 3:

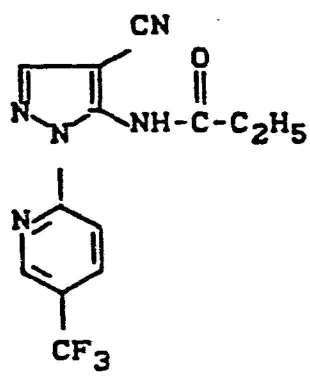

[Verfahren (c)]

Zu 2.5 g (0.01 Mol) 5-Amino-4-cyano-1-(5-trifluormethylpyrid-2-yl)-pyrazol in 30 ml Chloroform gibt man bei 0° bis 5°C unter Rühren tropfenweise zunächst 10 ml (10.6 g/ 0.11 Mol) Propionylchlorid und danach 1.8 ml (1.8 g/ 0.02 Mol) Pyridin in 15 ml Chloroform und rührt nach

beendeter Zugabe 16 Stunden bei Raumtemperatur. Zur Aufarbeitung gibt man 50 ml Ethanol zu und stellt mit konzentrierter wässriger Ammoniaklösung die Reaktionsmischung alkalisch, engt im Vakuum ein, nimmt den Rückstand in 100 ml Chloroform auf, wäscht mit Wasser, 2 normaler Salzsäure und wieder mit Wasser, trocknet über Natriumsulfat und entfernt das Lösungsmittel im Vakuum. Man erhält 2.6 g (84 % der Theorie) an 4-Cyano-5-propionamido-1-(5-trifluormethylpyrid-2-yl)-pyrazol vom Schmelzpunkt 130° - 132°C.

Beispiel 4:

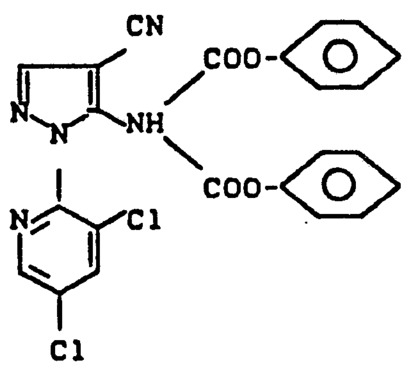

[Verfahren (c)]

Zu 7.6 g (0.03 Mol) 5-Amino-4-cyano-1-(3,5-dichlor-2-pyridyl)-pyrazol in 60 ml Chloroform gibt man zwischen 0° und 5°C zunächst 15.7 g (0.09 Mol) Chlorameisensäurephenylester und danach 7.1 g (0.09 Mol) Pyridin, rührt 15 Stunden bei Raumtemperatur, engt im Vakuum ein, verrührt den Rückstand mit Ether, saugt den so erhaltenen kristallinen Feststoff ab, wäscht mit Ether und Wasser nach, trocknet ihn bei 100°C und kristallisiert aus Toluol

um. Man erhält 9.8 g (66.1 % der Theorie) an 4-Cyano-5-[N,N-bis-(phenoxycarbonyl)-amino]-1-(3,5-dichlor-2-pyridyl)-pyrazol vom Schmelzpunkt 173° - 175°C.

Beispiel 5:

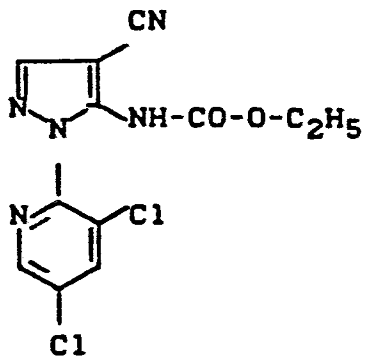

[Verfahren (c)]

Zu 12.7 g (0.05 Mol) 5-Amino-4-cyano-1-(3,5-dichlor-2-pyridyl)-pyrazol in 100 ml Pyridin gibt man unter Rühren und Eiskühlung zunächst 0.6 g (0.005 Mol) 4-(N,N-Dimethylamino)-pyridin und dann tropfenweise 81 g (0.5 Mol) Pyrokohlensäurediethylester und erwärmt nach beendeter Zugabe bis zum Ende der Kohlendioxid-Entwicklung auf 70°C. Zur Aufarbeitung engt man im Vakuum ein, kocht den Rückstand in 30 ml Ethanol auf, filtriert heiß und kühlt das Filtrat auf 0°C ab. Die so erhaltenen Kristalle lassen sich aus Ethanol/Petrolether (1:1) umkristallisieren. Man erhält 11 g (67.5 % der Theorie) an 4-Cyano-5-ethoxycarbonylamino-1-(3,5-dichlor-2-pyridyl)-pyrazol vom Schmelzpunkt 131° - 132°C.

In entsprechender Weise und gemäß den allgemeinen Herstellungsangaben erhält man die folgenden 5-Amino-4-cyano-1-pyridyl-pyrazole der allgemeinen Formel (I):

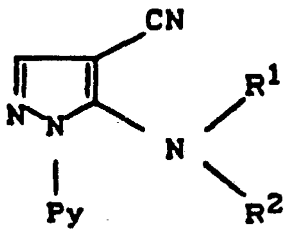

(I)

Tabelle 2:

| Bsp. Nr. | $R^1$ | $R^2$ | Py | Schmelzpunkt / °C |
|---|---|---|---|---|
| 6 | H | H | 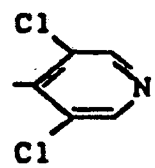 | 195-200 |
| 7 | $C_2H_5$-CO- | H | 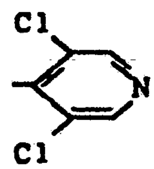 | 165-168 |
| 8 | H | H | 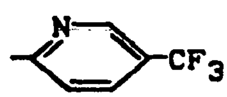 | 197-203 |
| 9 | $CH_3-(CH_2)_3-$ | H | 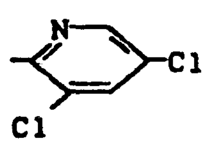 | 83 |

Tabelle 2 (Fortsetzung)

| Bsp. Nr. | $R^1$ | $R^2$ | Py | Schmelzpunkt / °C |
|---|---|---|---|---|
| 10 | $(CH_3)_2CH-$ | H | 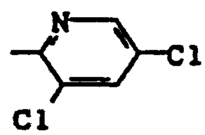 | Öl |
| 11 | $(CH_3O)_2CH-\overset{\displaystyle CH_3}{\overset{\vert}{C}}H-$ | H | 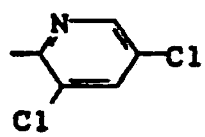 | 118-128 |
| 12 | $C_2H_5-CO-$ | $C_2H_5-CO-$ | 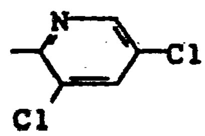 | Öl |
| 13 | $C_2H_5-CO-$ | H | 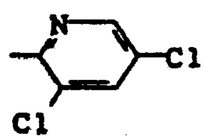 | 164-165 |
| 14 | $CH_3-CO-$ | H | 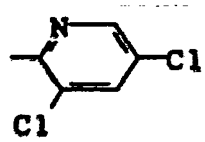 | 189-190 |
| 15 | $C_2H_5O-CO-$ | $C_2H_5O-CO-$ | 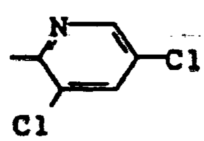 | 106-109 |
| 16 | $CH_3O-CO-$ | $CH_3O-CO-$ | 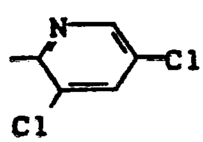 | 130-132 |

Tabelle 2 (Fortsetzung

| Bsp. Nr. | $R^1$ | $R^2$ | Py | Schmelz-punkt / °C |
|---|---|---|---|---|
| 17 | $CH_3O-CO-$ | H | 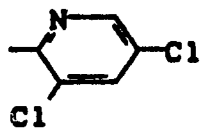 | 148-153 |
| 18 | $(CH_3)_2CH-CO-$ | H | 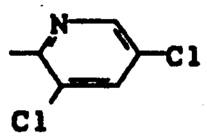 | Öl |
| 19 | $CH_3O-CO-CH(CH_3)-$ | H | 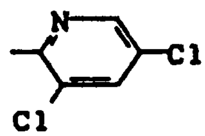 | 101 |

Anwendungsbeispiele:

In den folgenden Anwendungsbeispielen wurde die nachstehend aufgeführte Verbindung als Vergleichssubstanz eingesetzt:

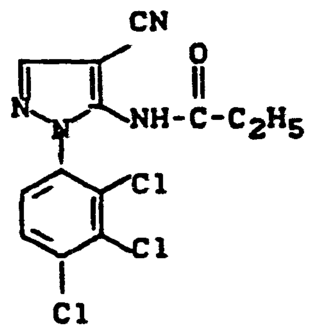

(A)

4-Cyano-5-propionamido-1-(2,3,4-trichlorphenyl)-pyrazol (bekannt aus DE-OS 32 26 513).

**Beispiel A**

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber der Vergleichssubstanz (A) zeigen in diesem Test z. B. die Verbindungen gemäß den Herstellungsbeispielen 5, 9, 11, 12 und 15.

**Beispiel B**

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine deutliche Überlegenheit in der Nutzpflanzenselektivität gegenüber der Vergleichssubstanz (A) zeigen in diesem Test z. B. die Verbindungen gemäß den Herstellungsbeispielen 5, 13, 14, 15 und 17.

Patentansprüche

1. 5-Amino-4-cyano-1-pyridyl-pyrazole der Formel (I),

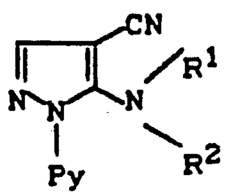

(I)

in welcher

$R^1$ für Wasserstoff, für jeweils gegebenenfalls substituiertes Alkyl, Alkenyl, Alkinyl oder Cycloalkyl oder für einen Rest -C-$R^3$ steht,
‖
X

$R^2$ für Wasserstoff, für jeweils gegebenenfalls substituiertes Alkyl, Alkenyl, Alkinyl oder Cycloalkyl oder für einen Rest -C-$R^3$ steht, wobei
‖
X

X für Sauerstoff oder Schwefel steht und

$R^3$ für Wasserstoff, Alkyl, Alkoxy, Alkoxyalkyl, Alkylthio, Alkylthioalkyl, Halogenalkyl, Alkenyl, Alkinyl, für jeweils gegebenenfalls substituiertes Cycloalkyl, Aryl, Aryloxy, Arylthio oder Arylamino, für Alkylamino oder für Dialkylamino steht

oder

$R^1$ und $R^2$ gemeinsam mit dem Stickstoffatom, an welches sie gebunden sind, für einen gegebenenfalls substituierten Heterocyclus der Formel

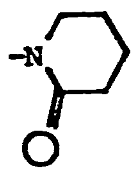 , 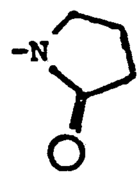 oder 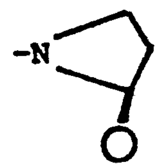

stehen und

Py für substituiertes Pyridyl steht, aber nicht für den 5-Nitro-2-pyridyl- oder den 3-Chlor-5-trifluormethyl-2-pyridyl-Rest steht.

2. 5-Amino-4-cyano-1-pyridyl-pyrazole der Formel (I)

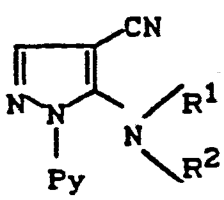

(I)

in welcher

$R^1$ für Wasserstoff, für jeweils gegebenenfalls einfach oder mehrfach, gleich oder verschieden substituiertes Alkyl mit 1 bis 8 Kohlenstoffatomen, Alkenyl oder Alkinyl mit jeweils 2 bis 8 Kohlenstoffatomen steht, wobei als Substituenten jeweils infrage kommen:
Halogen, Cyano, Nitro, Hydroxy, Carboxy, jeweils geradkettiges oder verzweigtes Alkoxy oder Alkoxycarbonyl mit jeweils 1 bis 6 Kohlenstoffatomen im Alkylteil, Aminocarbonyl, welches gegebenenfalls durch Alkyl, Alkoxy, Alkylsulfonyl, Alkenyl oder Alkinyl substituiert sein kann und wobei das Stickstoffatom des Aminocarbonylrestes auch Teil eines gesättigten 3- bis 7-gliedrigen Heterocyclus mit 1 oder 2 weiteren Heteroatomen, insbesondere Stickstoff, Sauerstoff oder Schwefel, sein kann:
für gegebenenfalls einfach oder mehrfach, gleich oder verschieden durch Halogen und/oder geradkettiges oder verzweigtes Alkyl mit 1 bis 4 Kohlenstoffatomen substituiertes Cycloalkyl mit 3 bis 7 Kohlenstoffatomen steht oder für einen Rest $-\underset{\underset{X}{\|}}{C}-R^3$ steht,

$R^2$ für Wasserstoff, für jeweils gegebenenfalls einfach oder mehrfach, gleich oder verschieden substituiertes Alkyl mit 1 bis 8 Kohlenstoffatomen, Alkenyl oder Alkinyl mit jeweils 2 bis 8 Kohlenstoffatomen steht, wobei als Substituenten jeweils infrage kommen:
Halogen, Cyano, Nitro, Hydroxy, Carboxy, jeweils geradkettiges oder verzweigtes Alkoxy oder Alkoxycarbonyl mit 1 bis 6 Kohlenstoffatomen im Alkylteil, Aminocarbonyl, welches gegebenenfalls durch Alkyl, Alkoxy, Alkylsulfonyl, Alkenyl oder Alkinyl substituiert sein kann und wobei das

Stickstoffatom des Aminocarbonylrestes auch Teil eines gesättigten 3- bis 7-gliedrigen Heterocyclus mit 1 oder 2 weiteren Heteroatomen, insbesondere Stickstoff, Sauerstoff oder Schwefel, sein kann:
für gegebenenfalls einfach oder mehrfach, gleich oder verschieden durch Halogen und/oder geradkettiges oder verzweigtes Alkyl mit 1 bis 4 Kohlenstoffatomen substituiertes Cycloalkyl mit 3 bis 7 Kohlenstoffatomen steht oder für einen Rest $-\underset{\underset{X}{\|}}{C}-R^3$ steht,

wobei

X für Sauerstoff oder Schwefel steht,

$R^3$ für Wasserstoff, für jeweils geradkettiges oder verzweigtes Alkyl, Alkoxy, Alkoxyalkyl, Alkylthio oder Alkylthioalkyl mit jeweils 1 bis 4 Kohlenstoffatomen in den einzelnen Alkylteilen, für geradkettiges oder verzweigtes Halogenalkyl mit 1 bis 4 Kohlenstoffatomen und 1 bis 9 gleichen oder verschiedenen Halogenatomen, für jeweils geradkettiges oder verzweigtes Alkenyl oder Alkinyl mit jeweils 2 bis 4 Kohlenstoffatomen, für gegebenenfalls einfach oder mehrfach, gleich oder verschieden substituiertes Cycloalkyl mit 3 bis 7 Kohlenstoffatomen, wobei als Substituenten Halogen, $C_1$-$C_4$-Alkyl oder

Halogen-$C_1$-$C_4$-alkyl. infrage kommen, für jeweils gegebenenfalls einfach oder mehrfach, gleich oder verschieden substituiertes Phenyl, Phenoxy, Phenylthio oder Phenylamino, wobei als Phenylsubstituenten Halogen, jeweils geradkettiges oder verzweigtes Alkyl oder Alkoxy mit jeweils 1 bis 4 Kohlenstoffatomen oder geradkettiges oder verzweigtes Halogenalkyl mit 1 bis 4 Kohlenstoffatomen und 1 bis 9 gleichen oder verschiedenen Halogenatomen infrage kommen, oder für jeweils geradkettiges oder verzweigtes Alkylamino oder Dialkylamino mit jeweils 1 bis 4 Kohlenstoffatomen in den einzelnen Alkylteilen steht,

oder

$R^1$ und $R^2$ gemeinsam mit dem Stickstoffatom, an welches sie gebunden sind, für einen jeweils gegebenenfalls einfach oder mehrfach, gleich oder verschieden durch geradkettiges oder verzweigtes Alkyl mit 1 bis 4 Kohlenstoffatomen substituierten Heterocyclus der Formel

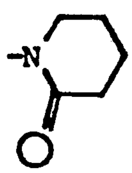 , 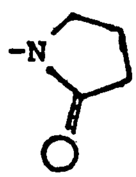 oder 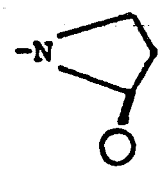

stehen

und

Py für jeweils einfach oder mehrfach, gleich oder verschieden substituiertes 2-Pyridyl, 3-Pyridyl oder 4-Pyridyl steht, wobei als Substituenten jeweils infrage kommen: Cyano, Nitro, Halogen, jeweils geradkettiges oder verzweigtes Alkyl, Alkoxy oder Alkoxycarbonyl mit 1 bis 4 Kohlenstoffatomen im Alkylteil, jeweils geradkettiges oder verzweigtes Halogenalkyl oder Halogenalkoxy mit jeweils 1 bis 4 Kohlenstoffatomen und jeweils 1 bis 9 gleichen oder verschiedenen Halogenatomen oder ein Rest $-S(O)_m-R^4$, wobei

$R^4$ für Amino, für jeweils geradkettiges oder verzweigtes Alkyl, Alkylamino oder Dialkylamino mit jeweils 1 bis 4 Kohlenstoffatomen in den einzelnen Alkylteilen oder für geradkettiges oder verzweigtes Halogenalkyl mit 1 bis 4 Kohlenstoffatomen und 1 bis 9 gleichen oder verschiedenen Halogenatomen steht und

m für eine Zahl 0, 1 oder 2 steht,

Py aber nicht für den 5-Nitro-2-pyridyl-Rest oder den 3-Chlor-5-trifluormethyl-2-pyridyl-Rest steht.

3. 5-Amino-4-cyano-1-pyridyl-pyrazole der Formel (I),

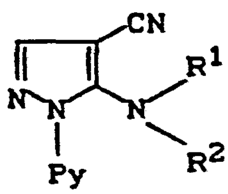

(I)

in welcher

$R^1$ für Wasserstoff, für jeweils gegebenenfalls ein- bis dreifach, gleich oder verschieden substituiertes Methyl, Ethyl, n- oder i-Propyl, n-, i-, s- oder t-Butyl, Allyl, Propenyl, Butenyl, Propargyl oder Butinyl steht, wobei als Substituenten jeweils infrage kommen: Fluor, Chlor, Brom, Iod, Cyano, Nitro, Hydroxy, Carboxy, Methoxy, Ethoxy, n- oder i-Propoxy, Methoxycarbonyl, Ethoxycarbonyl, n- oder i-Propoxycarbonyl, n-, i-, s- oder t-Butoxycarbonyl, Aminocarbonyl, N-Methylaminocarbonyl, N-Ethylaminocarbonyl, N,N-Dimethylaminocarbonyl, N,N-Diethylaminocarbonyl, N-Methyl-N-ethylaminocarbonyl, N-Methyl-N-propylaminocarbonyl, N-Methyl-N-methoxyaminocarbonyl, N-Methylsulfonylaminocarbonyl, N-Ethylsulfonylaminocarbonyl, N,N-Diallylaminocarbonyl, N-Methyl-N-propargylaminocarbonyl, Pyrrolidin-1-ylcarbonyl, Piperidin-1-ylcarbonyl, Morpholin-4-ylcarbonyl oder Perhydroazepin-1-ylcarbonyl;
für jeweils gegebenenfalls ein- bis fünffach gleich oder verschieden durch Chlor, Methyl oder Ethyl substituiertes Cyclopropyl, Cyclopentyl, Cyclohexyl oder Cycloheptyl steht oder

für einen Rest $-\underset{\underset{X}{\|}}{C}-R^3$ steht,

$R^2$ für Wasserstoff, für jeweils gegebenenfalls ein- bis dreifach, gleich oder verschieden substituiertes Methyl, Ethyl, n- oder i-Propyl, n-, i-, s- oder t-Butyl, Allyl, Propenyl, Butenyl, Propargyl oder Butinyl steht, wobei als Substituenten jeweils infrage kommen: Fluor, Chlor, Brom, Iod, Cyano, Nitro, Hydroxy, Carboxy, Methoxy, Ethoxy, n- oder i-Propoxy, Methoxycarbonyl, Ethoxycarbonyl, n- oder i-Propoxycarbonyl, n-, i-, s- oder t-Butoxycarbonyl, Aminocarbonyl, N-Methylaminocarbonyl, N-Ethylaminocarbonyl, N,N-Dimethylaminocarbonyl, N,N-Diethylaminocarbonyl, N-Methyl-N-ethylaminocarbonyl, N-Methyl-N-propylaminocarbonyl, N-Methyl-N- methoxyaminocarbonyl, N-Methylsulfonylaminocarbonyl, N-Ethylsulfonylaminocarbonyl, N,N-Diallylaminocarbonyl, N-Methyl-N-propargylaminocarbonyl, Pyrrolidin-1-ylcarbonyl, Piperidin-1-ylcarbonyl, Morpholin-4-ylcarbonyl oder Perhydroazepin-1-ylcarbonyl;

für jeweils gegebenenfalls ein- bis fünffach, gleich oder verschieden durch Chlor, Methyl oder Ethyl substituiertes Cyclopropyl, Cyclopentyl, Cyclohexyl oder Cycloheptyl steht oder für einen Rest $-\underset{\underset{X}{\|}}{C}-R^3$ steht, wobei

X für Sauerstoff oder Schwefel steht und

$R^3$ für Wasserstoff, Methyl, Ethyl, n- und i-Propyl, Methoxy, Ethoxy, Methoxymethyl, Ethoxymethyl, Methoxyethyl, Ethoxyethyl, Methylthio, Ethylthio, Methylthiomethyl, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 2-Bromethyl, Heptafluor-n-propyl, Allyl, Propenyl, Butenyl, Propargyl, für jeweils gegebenenfalls ein- bis vierfach, gleich oder verschieden durch Fluor, Chlor, Brom, Methyl oder Trifluormethyl substituiertes Cyclopropyl, Cyclopentyl oder Cyclohexyl, für jeweils gegebenenfalls ein- bis dreifach, gleich oder verschieden durch Methyl, Methoxy, Chlor oder Trifluormethyl substituiertes Phenyl, Phenoxy, Phenylthio oder Phenylamino steht,

oder

$R^1$ und $R^2$ gemeinsam mit dem Stickstoffatom, an welches sie gebunden sind, für einen jeweils gegebenenfalls ein- bis vierfach, gleich oder verschieden durch Methyl, Ethyl, n- oder i-Propyl substituierten Heterocyclus der Formel

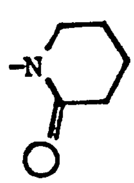 , 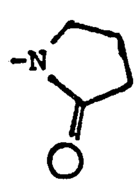 oder 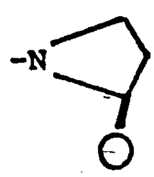

stehen und

Py für jeweils ein- bis vierfach, gleich oder verschieden substituiertes 2-Pyridyl, 3-Pyridyl oder 4-Pyridyl steht, wobei als Substituenten jeweils infrage kommen: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^4$, wobei

$R^4$ für Methyl, Ethyl, für Amino, Methylamino, Ethylamino, Dimethylamino, Diethylamino, Fluordichlormethyl, Difluormethyl, Tetrafluorethyl, Trifluorchlorethyl, Trichlormethyl oder Trifluormethyl steht und

m für eine Zahl 0, 1 oder 2 steht,

Py aber nicht für den 5-Nitro-2-pyridyl-Rest oder den 3-Chlor-5-trifluormethyl-2-pyridyl-Rest steht.

4. Verfahren zur Herstellung von 5-Amino-4-cyano-1-pyridyl-pyrazolen der Formel (I),

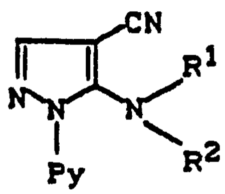

(I)

in welcher

$R^1$ für Wasserstoff, für jeweils gegebenenfalls substituiertes Alkyl, Alkenyl, Alkinyl oder Cycloalkyl oder für einen Rest $-C-R^3$ steht,
‖
X

$R^2$ für Wasserstoff, für jeweils gegebenenfalls substituiertes Alkyl, Alkenyl, Alkinyl oder Cycloalkyl oder für einen Rest $-C-R^3$ steht, wobei
‖
X

X für Sauerstoff oder Schwefel steht und

$R^3$ für Wasserstoff, Alkyl, Alkoxy, Alkoxyalkyl, Alkylthio, Alkylthioalkyl, Halogenalkyl, Alkenyl, Alkinyl, für jeweils gegebenenfalls substituiertes Cycloalkyl, Aryl, Aryloxy, Arylthio oder Arylamino, für Alkylamino oder für Dialkylamino steht

oder

$R^1$ und $R^2$ gemeinsam mit dem Stickstoffatom, an welches sie gebunden sind, für einen gegebenenfalls substituierten Heterocyclus der Formel

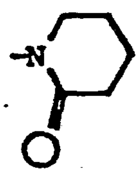 , 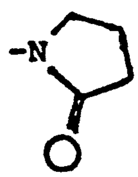 oder 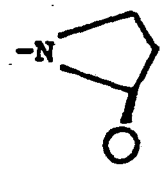

stehen und

Py für substituiertes Pyridyl steht, aber nicht für den 5-Nitro-2-pyridyl- oder den 3-Chlor-5-trifluormethyl-2-pyridyl-Rest steht,

dadurch gekennzeichnet, daß man

(a) die erfindungsgemäßen 5-Amino-4-cyano-1-pyridyl-pyrazole der Formel (I) erhält,

wenn man

5-Halogen-4-cyano-1-pyridyl-pyrazole der Formel (II)

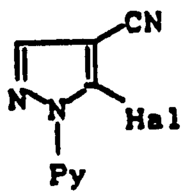

(II)

in welcher

Hal für Halogen steht und

Py die oben angegebene Bedeutung hat,

mit Aminoverbindungen der Formel (III),

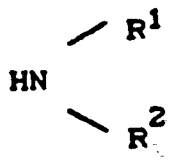

(III)

in welcher

$R^1$ und $R^2$ die oben angegebene Bedeutung haben,

gegebenenfalls in Gegenwart eines Verdünnungsmittels umsetzt,

oder daß man

(b) die erfindungsgemäßen 5-Amino-4-cyano-1-pyridyl-pyrazol-Derivate der Formel (Ib) erhält,

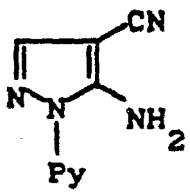

(Ib)

in welcher

Py die oben angegebene Bedeutung hat, wenn man

Pyridylhydrazine der Formel (IV),

$$Py-NH-NH_2 \qquad (IV)$$

in welcher

Py die oben angegebene Bedeutung hat,

mit Acrylnitrilderivaten der Formel (V),

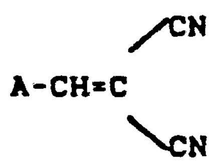

(V)

in welcher

A für Halogen, Hydroxy, Alkoxy oder Dialkylamino steht,

zunächst in einer 1. Stufe, gegebenenfalls in Gegenwart eines Verdünnungsmittels sowie gegebenenfalls in Gegenwart eines Reaktionshilfsmittels, umsetzt zu den Pyridylhydrazin-Derivaten der Formel (VI),

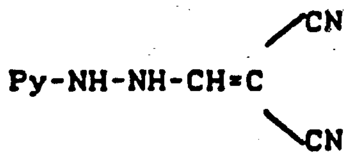

(VI)

in welcher

Py die oben angegebene Bedeutung hat,

und diese in einer 2. Stufe gegebenenfalls in Gegenwart eines Verdünnungsmittels und gegebenenfalls in Gegenwart eines Reaktionshilfsmittels cyclisiert,

oder daß man

(c) die erfindungsgemäßen 5-Amino-4-cyano-1-pyridyl-pyrazol-Derivate der Formel (Ic) erhält,

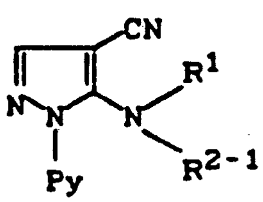

(Ic)

in welcher

$R^{2-1}$ für jeweils gegebenenfalls substituiertes Alkyl, Alkenyl, Alkinyl oder Cycloalkyl oder für einen Rest $-\underset{\underset{X}{\|}}{C}-R^3$ steht und

$R^1$, $R^3$, Py und X die oben angegebene Bedeutung haben,
wenn man

5-Amino-4-cyano-1-pyridyl-pyrazole der Formel (Ik),

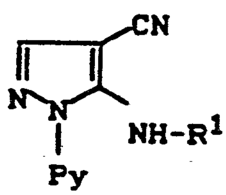

(Ik)

in welcher

$R^1$ und Py die oben angegebene Bedeutung haben,
mit Verbindungen der Formel (VII)

$$R^{2-1}-A^1 \qquad (VII)$$

in welcher

$R^{2-1}$ für jeweils gegebenenfalls substituiertes Alkyl, Alkenyl, Alkinyl oder Cycloalkyl oder für einen Rest -C-$R^3$ steht, wobei
||
X

$R^3$ und X die oben angegebene Bedeutung haben und

$A^1$ für eine elektronenziehende Abgangsgruppe steht,

gegebenenfalls in Gegenwart eines Verdünnungsmittels und gegebenenfalls in Gegenwart eines Säurebindemittels umsetzt,

oder daß man

(d) die erfindungsgemäßen 5-Amino-4-cyano-1-pyridyl-pyrazole der Formel (Id) erhält,

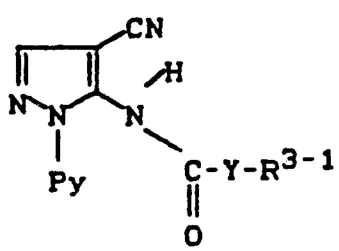

(Id)

in welcher

$R^{3-1}$ für Alkyl oder für gegebenenfalls substituiertes Aryl steht,

Y für Sauerstoff, Schwefel, eine (-NH-)- oder eine (>N-Alkyl)-Gruppe steht und

Py die oben angegebene Bedeutung hat,

wenn man

5-[N,N-Bis-(phenoxycarbonyl)-amino]-1-pyridyl-pyrazole der Formel (I1)

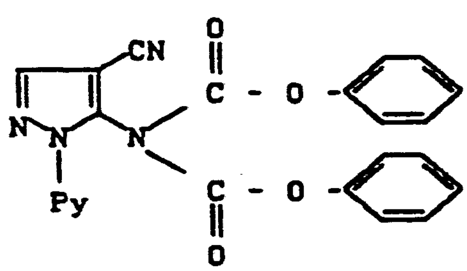

(I1)

in welcher

Py die oben angegebene Bedeutung hat,

mit Verbindungen der Formel (VIII),

$R^{3-1}$-Y-H (VIII)

in welcher

$R^{3-1}$ für Alkyl oder für gegebenenfalls substituiertes Aryl steht und

Y für Sauerstoff, Schwefel, eine (-NH-)- oder eine (>N-Alkyl)-Gruppe steht,

gegebenenfalls in Gegenwart eines Verdünnungsmittels und gegebenenfalls in Gegenwart eines basischen Reaktionshilfsmittels umsetzt,

oder daß man

(e) die erfindungsgemäßen 5-Amino-4-cyano-1-pyridyl-pyrazol-Derivate der Formel (Ie) erhält,

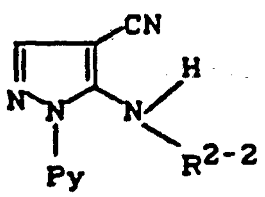

(Ie)

in welcher

$R^{2-2}$ für jeweils gegebenenfalls substituiertes Alkyl, Alkenyl, Alkinyl oder Cycloalkyl steht und

Py die oben angegebene Bedeutung hat,

wenn man

5-(N-Acylamino)-4-cyano-1-pyridyl-pyrazole der Formel (Im)

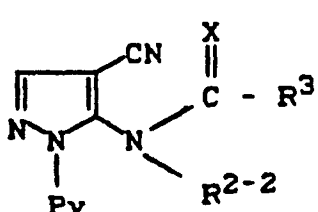

(Im)

in welcher

$R^3$, $R^{2-2}$, X und Py die oben angegebene Bedeutung haben,

mit Säuren oder Basen, gegebenenfalls in Gegenwart eines Verdünnungsmittels deacyliert,

oder daß man

(f) die erfindungsgemäßen 5-Amino-4-cyano-1-pyridyl-pyrazol-Derivate der Formel (If) erhält,

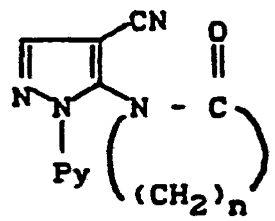

(If)

in welcher

n für 2, 3 oder 4 steht und

Py die oben angegebene Bedeutung hat,

wenn man

5-Halogenacylamino-4-cyano-pyrazole der Formel (In)

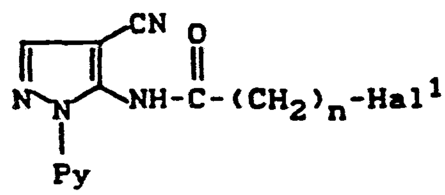

(In)

in welcher

$Hal^1$ für Halogen steht,

n für 2, 3 oder 4 steht und

Py die oben angegebene Bedeutung hat,

gegebenenfalls in Gegenwart eines Verdünnungsmittels und gegebenenfalls in Gegenwart eines basischen Reaktionshilfsmittels intramolekular cyclisiert,

oder daß man

(g) die erfindungsgemäßen 5-Amino-4-cyano-1-pyridyl-pyrazol-Derivate der Formel (Ig) erhält,

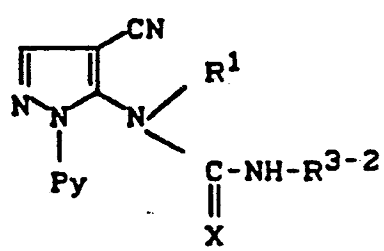

(Ig)

in welcher

$R^{3-2}$ für Alkyl oder für gegebenenfalls substituiertes Aryl steht,

X für Sauerstoff oder Schwefel steht und

$R^1$ und Py die oben angegebene Bedeutung haben,

wenn man

5-Amino-4-cyano-1-pyridyl-pyrazole der Formel (Ik),

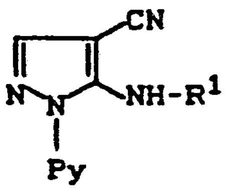

(Ik)

in welcher

$R^1$ und Py die oben angegebene Bedeutung haben,

mit Iso-(thio)-cyanaten der Formel (IX),

$R^{3-2}$-N=C=X (IX)

in welcher

$R^{3-2}$ und X die oben angegebene Bedeutung haben,

gegebenenfalls in Gegenwart eines Verdünnungsmittels und gegebenenfalls in Gegenwart eines Reaktionshilfsmittels umsetzt.

5. Herbizide Mittel, gekennzeichnet durch einen Gehalt an mindestens einem 5-Amino-4-cyano-1-pyridyl-pyrazol der Formel (I) gemäß den Ansprüchen 1 bis 4.

6. Verfahren zur Bekämpfung von Unkräutern, dadurch gekennzeichnet, daß man ein 5-Amino-4-cyano-1-pyridyl-pyrazol der Formel (I) gemäß den Ansprüchen 1 bis 4

auf Unkräuter und/oder ihren Lebensraum einwirken läßt.

7. Verwendung von 5-Amino-4-cyano-1-pyridyl-pyrazolen der Formel (I) gemäß den Ansprüchen 1 bis 4 zur Bekämpfung von unerwünschtem Pflanzenwuchs.

8. Verfahren zur Herstellung von herbiziden Mitteln, dadurch gekennzeichnet, daß man 5-Amino-4-cyano-1-pyridyl-pyrazole der Formel (I) gemäß den Ansprüchen 1 bis 4 mit Streckmitteln und/oder oberflächenaktiven Mitteln vermischt.